# EUROPÄISCHE PATENTSCHRIFT

(11) **EP 4 054 447 B1**
(45) Veröffentlichungstag und Bekanntmachung des Hinweises auf die Patenterteilung: **13.03.2024**
(21) Anmeldenummer: 20803530.3
(22) Anmeldetag: 06.11.2020
(51) Int. Cl.: A61B 17/29, A61B 17/122, A61B 17/128

(54) **MEDIZINISCHES SCHIEBESCHAFTINSTRUMENT**
MEDICAL SLIDING SHAFT INSTRUMENT
INSTRUMENT MÉDICAL À TIGE COULISSANTE

(30) Priorität: 08.11.2019 DE 102019130223
(43) Veröffentlichungstag der Anmeldung: 14.09.2022
(73) Patentinhaber: Aesculap AG, 78532 Tuttlingen (DE)
(72) Erfinder: MATTES, Richard, 78589 Dürbheim (DE); PLEIL, Thomas, 78073 Bad Dürrheim (DE)
(74) Vertreter: Hoeger, Stellrecht & Partner Patentanwälte mbB
(86) Internationale Anmeldenummer: PCT/EP2020/081251
(87) Internationale Veröffentlichungsnummer: WO 2021/089763

(56) Entgegenhaltungen:
- DE-B3- 10 321 854
- DE-U1- 29 800 876
- US-A1- 2003 191 478
- US-A9- 2016 331 369

## Beschreibung

Die vorliegende Erfindung betrifft ein medizinisches Schiebeschaftinstrument mit einem Werkzeugelementträger, einem rohrförmigen, den Werkzeugelementträger umgebenden und eine Verschieberichtung definierenden Schiebeschaft und einer Betätigungseinrichtung zum Bewegen des Schiebeschafts relativ zum Werkzeugelementträger in distaler Richtung, wobei am distalen Ende des Werkzeugelementträgers mindestens ein Werkzeugelement angeordnet oder beweglich gelagert ist, wobei das mindestens eine Werkzeugelement mit einem distalen Endbereich des Schiebeschafts zusammenwirkend angeordnet oder ausgebildet ist derart, dass in Folge einer Bewegung des Schiebeschafts in distaler Richtung das mindestens eine Werkzeugelement bewegt wird, wobei das Instrument ferner eine Sperreinrichtung umfasst zum Blockieren einer Bewegung des Schiebeschafts relativ zum Werkzeugelementträger in mindestens einer Vorschubstellung, wobei der Schiebeschaft in der mindestens einen Vorschubstellung gegenüber einer Grundstellung, in welcher der Schiebeschaft relativ zum Werkzeugelementträger maximal weit in proximaler Richtung verschoben ist, in distaler Richtung verschoben ist, wobei die Sperreinrichtung eine Freigabestellung, in welcher der Schiebeschaft relativ zum Werkzeugelementträger, insbesondere in proximaler Richtung, verschiebbar ist und eine Sperrstellung definiert, in welcher eine Bewegung des Schiebeschafts relativ zum Werkzeugelementträger in proximaler und/oder distaler Richtung blockiert ist, wobei die Sperreinrichtung mindestens ein erstes Sperrelement und mindestens ein zweites Sperrelement umfasst, welche in der Sperrstellung kraft- und/oder formschlüssig in Eingriff und in der Freigabestellung außer Eingriff stehen.

Medizinische Schiebeschaftinstrumente sind beispielsweise in der DE 101 55 734 C1 oder in der DE 103 21 854 B3 offenbart. Die in diesen Druckschriften beschriebenen Schiebeschaftinstrumente können optional mit einer Sperre in Form einer eingangs beschriebenen Sperreinrichtung ausgestattet sein, um den Schiebeschaft relativ zum Werkzeugelementträger in einer Vorschubstellung zu halten, beispielsweise temporär. Dies bedeutet, dass eine Bewegung des Schiebeschafts relativ zum Werkzeugelementträger in distaler und/oder proximaler Richtung blockiert wird, wenn die Sperreinrichtung aktiv ist, also die Sperrstellung einnimmt.

Herkömmliche Sperreinrichtungen umfassen beispielsweise zwei an Branchen der Betätigungseinrichtung abstehende Sperrelemente, die üblicherweise aus einem dünnen Blech ausgebildet und an die Branchen angeschweißt sind. Derartige Sperreinrichtungen sind aufgrund ihres Aufbaus reparaturanfällig und können durch eine nicht sachgemäße Aufbereitung und Handhabung deformiert werden, wodurch es zu Fehlfunktionen der Sperreinrichtung kommen kann.

Die DE 298 00 876 U1 betrifft ein chirurgisches Instrument. Aus der US 2016/0331369 A9 ist ein Nadeltreiber bekannt. Ein chirurgisches Werkzeug mit einem distalen Ratschenmechanismus ist in der US 2003/0191478 A1 beschrieben.

Es ist daher eine Aufgabe der vorliegenden Erfindung, ein medizinisches Schiebeschaftinstrument der eingangs beschriebenen Art so zu verbessern, dass es einfach und sicher handhabbar ist.

Diese Aufgabe wird bei einem medizinischen Schiebeschaftinstrument der eingangs beschriebenen Art erfindungsgemäß dadurch gelöst, dass das mindestens eine zweite Sperrelement am Schiebeschaft angeordnet oder ausgebildet ist und dass das mindestens eine erste Sperrelement von der Freigabestellung durch eine Bewegung auf den Schiebeschaft hin in die Sperrstellung überführbar ist.

Die vorgeschlagene Weiterbildung ermöglicht es insbesondere, das Schiebeschaftinstrument in der mindestens einen Vorschubstellung zu sperren, also beispielsweise eine Bewegung des Schiebeschafts relativ zum Werkzeugelementträger in proximaler Richtung zu blockieren. Die Anordnung des mindestens einen zweiten Sperrelements am Schiebeschaft oder die Ausbildung desselben direkt am Schiebeschaft vereinfacht den Aufbau des Instruments. So kann das mindestens eine zweite Sperrelement zum Beispiel einstückig im Sinne von monolithisch mit dem Schiebeschaft ausgebildet werden. Dies ist bei herkömmlich eingesetzten Sperreinrichtungen nicht möglich. So lässt sich die Stabilität des Schiebeschaftinstruments verbessern und damit auch dessen Handhabbarkeit. Das Überführen des Schiebeschaftinstruments von der Freigabestellung der Sperreinrichtung in die Sperrstellung wird durch eine Bewegung des mindestens einen ersten Sperrelements auf den Schiebeschaft hin erreicht. Dies ermöglicht insbesondere eine für den Benutzer besonders einfache und sichere Handhabung, da er mit dem mindestens einen ersten Sperrelement direkt am Schiebeschaft angreift, um das Schiebeschaftinstrument zu sperren. Insbesondere kann ein solches Schiebeschaftinstrument ausgebildet werden, ohne dass die Sperrelemente an der Betätigungseinrichtung angeordnet oder ausgebildet werden müssen. Dadurch kann insbesondere die Wahrscheinlichkeit einer versehentlichen Betätigung der Sperreinrichtung minimiert werden. Mit einer solchen Sperreinrichtung ist es insbesondere möglich, das mindestens eine Werkzeugelement, beispielsweise zwei Werkzeugelemente, in einer definierten, teilweise geschlossenen Stellung zu halten. Ist das Schiebeschaftinstrument in Form einer Clipanlegezange ausgebildet, kann so insbesondere ein kleiner Aneurysmenclip definiert mit einem ein wenig geöffneten Maul sicher gehalten und einem Operateur von einer ihm assistierenden Person das Schiebeschaftinstrument mit einem mit diesem gehaltenen Clip angereicht werden. Durch die vorgeschlagene Anordnung der Sperrelemente ist insbesondere eine bezogen auf eine die Verschieberichtung enthaltende Ebene spiegelsymmetrische Anordnung oder Ausbildung der Sperreinrichtung möglich. Dies gestattet es insbesondere, das Schiebeschaftinstrument und so auch die Sperreinrichtung sowohl für Links- als auch für Rechtshänder sicher nutzbar zu machen.

Vorteilhaft ist es, wenn das mindestens eine zweite Sperrelement und der Schiebeschaft einstückig ausgebildet sind. Insbesondere können sie monolithisch ausgebildet sein. Auf diese Weise lässt sich das Schiebeschaftinstrument deutlich einfacher herstellen, wodurch eine Produktivität und eine Prozesssicherheit bei der Herstellung des Schiebeschaftinstruments verbessert werden können. Durch die einstückige Ausbildung des zweiten Sperrelements und des Schiebeschafts kann die Sperreinrichtung im Vergleich zu herkömmlichen Sperreinrichtungen deutlich weniger reparaturanfällig ausgebildet werden, was zu einer höheren Standzeit und damit auch zu einer längeren Lebensdauer des Schiebeschaftinstruments führt.

Günstig ist es, wenn das mindestens eine erste Sperrelement in einer Richtung quer, insbesondere senkrecht, zur Verschieberichtung von der Freigabestellung in die Sperrstellung bewegbar ist. Eine solche Ausgestaltung ermöglicht es insbesondere, mit dem mindestens einen ersten Sperrelement direkt am Schiebeschaft anzugreifen und eine Verschiebung des Schiebeschafts relativ zum Werkzeugelementträger in proximaler und/oder distaler Richtung blockieren.

Vorteilhaft ist es, wenn das mindestens eine erste Sperrelement am Schiebeschaftinstrument und relativ zum Werkzeugelementträger in der Verschieberichtung unbeweglich oder im Wesentlichen unbeweglich angeordnet oder ausgebildet ist. Dies ist insbesondere so zu verstehen, dass das mindestens eine erste Sperrelement nicht in beispielsweise einer axialen Richtung, die vom Werkzeugelementträger oder vom Schiebeschaft definiert wird, bewegbar ist, sondern lediglich quer hierzu. Im Wesentlichen unbeweglich bedeutet, dass beispielsweise ein sich parallel zur Verschieberichtung erstreckender Hebel beziehungsweise Hebelarm, der vom ersten Sperrelement umfasst ist oder an dem das mindestens eine erste Sperrelement angeordnet oder ausgebildet ist, in der Verschieberichtung im Wesentlichen unbeweglich ist, wenn der Hebel mit einem freien Ende in Richtung auf den Schiebeschaft verschwenkt oder verformt werden kann, um die Sperreinrichtung von der Freigabestellung in die Sperrstellung zu überführen und umgekehrt.

Die Handhabung des Schiebeschaftinstruments kann für einen Anwender in gewohnter Weise realisiert werden, wenn die Betätigungseinrichtung zwei relativ zueinander bewegbare, Betätigungselemente umfasst. Insbesondere können die zwei Betätigungselemente verschwenkbar relativ zueinander angeordnet oder ausgebildet sein.

Für eine besonders gefühlvolle Handhabung des Schiebeschaftinstruments ist es vorteilhaft, wenn die zwei Betätigungselemente bei maximal in proximaler Richtung verschobenem Schiebeschaft maximal weit voneinander entfernt und zum Bewegen des Schiebeschafts in distaler Richtung aufeinander zu bewegbar sind. Die Betätigungselemente werden also aufeinander zu bewegt, das heißt gegeneinandergedrückt, um den Schiebeschaft in distaler Richtung zu bewegen. Die Grundstellung des mindestens einen Werkzeugelements wird wie bereits erwähnt dadurch definiert, dass der Schiebeschaft relativ zum Werkzeugelementträger maximal weit in proximaler Richtung verschoben ist.

Besonders einfach lässt sich das Schiebeschaftinstrument ausbilden, wenn die zwei Betätigungselemente jeweils um eine Schwenkachse verschwenkbar sind, welche quer zur Verschieberichtung verläuft. Insbesondere können die Schwenkachsen senkrecht zur Verschieberichtung verlaufen. Beispielsweise können die zwei Betätigungselemente unabhängig voneinander verschwenkbar gelagert sein, sodass sie jeweils eine Schwenkachse definieren. Diese können parallel verlaufen, oder, bei gemeinsamer Anlenkung, auch zusammenfallen.

Gemäß einer weiteren bevorzugten Ausführungsform der Erfindung kann vorgesehen sein, dass das Schiebeschaftinstrument eine Vorspanneinrichtung umfasst, zum Ausüben einer in proximaler Richtung wirkenden vorspannenden Kraft auf den Schiebeschaft. Eine solche Vorspanneinrichtung ermöglicht es insbesondere, den Schiebeschaft bei unbetätigtem Instrument, also wenn keine Kräfte durch einen Anwender auf die Betätigungseinrichtung ausgeübt werden und die Sperreinrichtung die Freigabestellung einnimmt, relativ zum Werkzeugelementträger maximal weit in proximaler Richtung zu verschieben. In dieser Stellung kann beispielsweise das mindestens eine Werkzeugelement seine eine Extremstellung einnehmen. Wenn beispielsweise zwei Werkzeugelemente vorgesehen sind, können diese dann maximal weit geöffnet oder maximal weit geschlossen sein, je nachdem, für welchen Anwendungszweck das Schiebeschaftinstrument vorgesehen sein soll.

Vorteilhaft ist es, wenn die Vorspanneinrichtung mindestens ein Vorspannelement umfasst und wenn die zwei Betätigungselemente entgegen der Wirkung des mindestens einen Vorspannelements aufeinander zu bewegbar sind. Eine derartige Ausgestaltung ermöglicht es insbesondere, das Schiebeschaftinstrument derart auszubilden, dass eine definierte Betätigungskraft zum Verschieben des Schiebeschafts in distaler Richtung erforderlich ist. Zudem ermöglicht es eine solche Vorspanneinrichtung, das Schiebeschaftinstrument selbsttätig wieder in die Grundstellung zu überführen, wenn die Sperreinrichtung unbetätigt ist, also die Freigabestellung einnimmt.

Günstigerweise umfasst das mindestens eine zweite Sperrelement eine in proximaler Richtung weisende Anschlagfläche. Dies ermöglicht es insbesondere, eine Bewegung des Schiebeschafts relativ zum Werkzeugelementträger in proximaler Richtung zu blockieren, wenn das mindestens eine erste Sperrelement an der Anschlagfläche angreift. Umfasst das Schiebeschaftinstrument eine Vorspanneinrichtung, kann diese beispielsweise das mindestens eine erste Sperrelement, das auf den Schiebeschaft hin vorgeschoben ist und an der Anschlagfläche anliegt, und das mit diesem zusammenwirkende zweite Sperrelement kraftschlüssig in Eingriff halten.

Um ein versehentliches Freigeben des Schiebeschaftinstruments, also das Überführen der Sperreinrichtung von der Sperrstellung in die Freigabestellung zu verhindern, ist es günstig, wenn das mindestens eine zweite Sperrelement eine Hinterschneidung aufweist und wenn die Hinterschneidung mindestens teilweise von der Anschlagfläche begrenzt ist. Die Ausbildung des mindestens einen zweiten Sperrelements kann so auf einfache Weise realisiert werden. Wenn dieses mindestens teilweise in die Hinterschneidung eingreift, bedarf es zum Lösen der Sperreinrichtung zunächst einer Bewegung des Schiebeschafts in distaler Richtung, um die Sperreinrichtung freizugeben. So kann ein versehentliches Öffnen oder Schließen von zwei zusammenwirkenden Werkzeugelementen am distalen Ende des Werkzeugelementträgers insbesondere beim Anreichen oder Übergeben des Schiebeschaftinstruments an einen Operateur von einer dieser ihm assistierenden Person verhindert werden.

Auf einfache Weise lässt sich das Schiebeschaftinstrument ausbilden, wenn die Anschlagfläche in Richtung auf den Schiebeschaft hin weisend geneigt ist. Eine solche Anschlagfläche kann auf einfache Weise einstückig am Schiebeschaft ausgebildet werden.

Um eine sichere Funktion der Sperreinrichtung erreichen zu können, ist es günstig, wenn ein Neigungswinkel zwischen der Anschlagfläche und der Verschieberichtung in einem Bereich von etwa 88° bis etwa 60° liegt. Eine derart geformte Hinterschneidung lässt sich noch einfach herstellen und zudem auch einfach und sicher reinigen.

Vorteilhaft ist es, wenn das mindestens eine zweite Sperrelement in Form eines vom Schiebeschaft in radialer Richtung weisenden Rückhaltevorsprungs oder in Form einer vom Schiebeschaft weg weisend geöffnete Rückhaltenut ausgebildet ist. Sowohl der Rückhaltevorsprung als auch die Rückhaltenut können insbesondere mit in proximaler Richtung weisenden Anschlagflächen ausgebildet werden. Sie lassen sich beide auf einfache Weise an einem Schiebeschaft anordnen oder ausbilden.

Vorzugsweise umgibt das mindestens eine zweite Sperrelement den Schiebeschaft ringförmig. Insbesondere ist das mindestens eine zweite Sperrelement in sich geschlossen ausgebildet. So können beispielsweise eine Rückhaltenut in Form einer Ringnut oder ein Rückhaltevorsprung in Form eines Ringvorsprungs am Schiebeschaft ausgebildet werden. Dies ermöglicht es insbesondere, die Sperreinrichtung unabhängig von einer Verdrehstellung des Schiebeschafts relativ zum Werkzeugelementträger um die Verschieberichtung oder eine von diesen definierte Längsrichtung durch einen sicheren Eingriff des mindestens einen ersten Sperrelements und des mindestens einen zweiten Sperrelements von der Freigabestellung in die Sperrstellung zu überführen.

Gemäß einer weiteren bevorzugten Ausführungsform der Erfindung kann vorgesehen sein, dass die Sperreinrichtung eine Rückstelleinrichtung umfasst derart, dass das mindestens eine erste Sperrelement entgegen der Wirkung der Rückstelleinrichtung von der Freigabestellung in die Sperrstellung bewegbar ist. Diese Ausgestaltung erfordert eine aktive Betätigung der Sperreinrichtung entgegen der Wirkung der Rückstelleinrichtung. So kann ein versehentliches Betätigen der Sperreinrichtung vermieden werden. Ferner ist es insbesondere möglich, die Sperreinrichtung derart auszubilden, dass sie automatisch von der Sperrstellung in die Freigabestellung übergeht, wenn das mindestens eine erste Sperrelement und das mindestens eine zweite Sperrelement außer Eingriff gebracht werden. Ist beispielsweise das mindestens eine zweite Sperrelement mit einer Hinterschneidung ausgebildet, welche in proximaler Richtung weisend geöffnet ist, kann die Sperreinrichtung automatisch gelöst werden, indem der Schiebeschaft relativ zum Werkzeugelementträger etwas in distaler Richtung vorgeschoben wird. Die Sperrelemente gelangen dann außer Eingriff und die Rückstelleinrichtung bewegt das mindestens eine erste Sperrelement in seine Ausgangsstellung zurück, beispielsweise in die Freigabestellung, in welcher die Sperrelemente außer Eingriff stehen.

Die Rückstelleinrichtung lässt sich auf einfache Weise ausbilden, wenn sie mindestens ein Rückstellelement umfasst, welches mit dem mindestens einen ersten Sperrelement zusammenwirkend angeordnet oder ausgebildet ist. Beispielsweise kann das mindestens eine Rückstellelement am Sperrelement angreifen oder von diesem umfasst sein.

Auf einfache Weise lässt sich das Schiebeschaftinstrument ausbilden, wenn das mindestens eine Rückstellelement ein Federelement umfasst. Insbesondere kann es Federelement in Form einer Schraubenfeder oder einer Blattfeder ausgebildet sein.

Günstig ist es, wenn das mindestens eine erste Sperrelement einen in Richtung auf den Schiebeschaft hin weisenden Sperrvorsprung umfasst, welcher in der Sperrstellung mit dem mindestens einen zweiten Sperrelement in Eingriff steht. So kann das mindestens eine erste Sperrelement optimal an den Schiebeschaft und das an diesem angeordnete oder ausgebildete zweite Sperrelement angepasst werden.

Vorzugsweise umfasst das mindestens eine erste Sperrelement eine Rastnase, welche in der Sperrstellung formschlüssig oder im Wesentlichen formschlüssig in die Hinterschneidung am mindestens einen zweiten Sperrelement eingreift. So lässt sich auf einfache Weise eine Verriegelung der Sperreinrichtung in der Sperrstellung erreichen, und zwar insbesondere dann, wenn das Schiebeschaftinstrument eine Vorspanneinrichtung umfasst zum Ausüben einer in proximaler Richtung wirkenden vorspannenden Kraft auf den Schiebeschaft. Dies ermöglicht es insbesondere, die Rastnase, die in die Hinterschneidung eingreift, unter Vorspannung in dieser Eingriffsstellung zu halten, und damit die Sperreinrichtung in der Sperrstellung.

Um insbesondere eine Bewegung des Schiebeschafts relativ zum Werkzeugelementträger in proximaler Richtung einfach und sicher zu verhindern, ist es vorteilhaft, wenn die Rastnase in distaler Richtung weisend angeordnet oder ausgebildet ist. Insbesondere kann die Rastnase am Sperrvorsprung angeordnet oder ausgebildet sein.

Günstig ist es, wenn das mindestens eine erste Sperrelement einen Hebelarm umfasst und wenn der Sperrvorsprung an einem freien Ende des Hebelarms angeordnet oder ausgebildet ist. Der Sperrvorsprung kann so beispielsweise in Richtung auf den Schiebeschaft hin bewegt werden, wenn der Hebelarm mit seinem freien Ende in Richtung auf den Schiebeschaft verschwenkt oder verformt wird.

Für eine einfache und sichere Handhabung des Schiebeschaftinstruments ist es günstig, wenn der Hebelarm in distaler Richtung weisend am Instrument angeordnet oder ausgebildet ist. Dies ermöglicht insbesondere auf einfache Weise eine Anordnung einer in distaler Richtung weisenden Rastnase am freien Ende des Hebelarms, beispielsweise an dem am freien Ende des Hebelarms angeordneten Sperrvorsprung.

Vorteilhaft ist es, wenn der Hebelarm proximalseitig am Instrument festgelegt ist. Insbesondere kann er um eine Hebelarmschwenkachse verschwenkbar gelagert sein, welche insbesondere quer, beispielsweise senkrecht, zur Verschieberichtung verläuft. Diese Ausgestaltung ermöglicht es insbesondere auf einfache Weise das mindestens eine erste Sperrelement in Form einer Sperrklinke auszubilden. Der Hebelarm kann proximalseitig auch einstückig am Schiebeschaftinstrument, also monolithisch, mit diesem ausgebildet sein. Auf diese Weise kann eine zusätzliche Lagerung des Hebelarms vermieden werden. Dies vereinfacht den Aufbau des Instruments und damit auch dessen Stabilität.

Vorzugsweise umfasst der Hebelarm das mindestens eine Rückstellelement oder bildet dieses. Beispielsweise kann der Hebelarm ein federelastisches Blattfederelement bilden, an dessen freiem Ende der Sperrvorsprung angeordnet ist. Ein in proximaler Richtung weisendes Ende des Hebelarms kann am Schiebeschaftinstrument angeordnet sein.

Vorzugsweise umfasst das mindestens eine erste Sperrelement einen quer zur Verschieberichtung verlaufenden Sperrelementabschnitt. Beispielsweise kann der Sperrelementabschnitt derart ausgebildet und angeordnet sein, dass er in der Sperrstellung mit dem mindestens einen zweiten Sperrelement zusammenwirkt.

Günstig ist es, wenn das mindestens eine Rückstellelement den Sperrelementabschnitt umgebend angeordnet oder ausgebildet ist. Dies ermöglicht es insbesondere, das mindestens eine erste Sperrelement auf einfache Weise in Form eines Ventildrückers auszubilden.

Günstig ist es, wenn der Sperrvorsprung an einem auf den Schiebeschaft hin weisenden Ende des Sperrelementabschnitts angeordnet oder ausgebildet ist. So kann also insbesondere nur der Teil des Sperrelementabschnitts, welcher mit dem mindestens einen zweiten Sperrelement zusammenwirkt oder in dieses eingreift, den Sperrvorsprung definieren.

Vorteilhaft ist es, wenn das Schiebeschaftinstrument eine Sicherungseinrichtung umfasst zum Sichern der Sperreinrichtung in der Sperrstellung. Mit einer solchen Sicherungseinrichtung kann insbesondere vermieden werden, dass das Schiebeschaftinstrument selbsttätig von der Sperrstellung in die Freigabestellung übergehen kann. Zum Lösen der Sperreinrichtung muss daher zunächst die Sicherungseinrichtung entsichert werden.

Auf einfache Weise lässt sich das Schiebeschaftinstrument ausbilden, wenn die Sicherungseinrichtung das mindestens eine erste Sperrelement und das mindestens eine zweite Sperrelement umfasst, welche derart angeordnet oder ausgebildet sind, dass die Sperreinrichtung nur durch eine Bewegung des Schiebeschafts in distaler Richtung entsicherbar ist. Die Sicherungseinrichtung lässt sich also insbesondere in die Sperreinrichtung integrieren, beispielsweise durch besondere Ausgestaltung der Sperrelemente. Diese können insbesondere in der beschriebenen Weise ausgebildet sein, sodass die Sperrstellung in die Freigabestellung überführbar ist, wenn der Schiebeschaft zunächst etwas in distaler Richtung relativ zum Werkzugelementträger bewegt wird. Dies kann durch eine besondere Ausgestaltung der Sperrelemente erreicht werden, welche ein einfaches außer Eingriff Bringen nur durch eine Bewegung relativ zueinander in radialer Richtung nicht ermöglichen.

Die Sicherungseinrichtung lässt sich auf einfache Weise ausbilden, wenn sie die Hinterschneidung und die Rastnase umfasst. Wenn diese derart ausgebildet sind, dass sie in distaler beziehungsweise proximaler Richtung weisend wirken, bilden sie zwei zusammenwirkende Sicherungselemente, die von der Sicherungseinrichtung umfasst sind und die nur durch eine Relativbewegung des Schiebeschafts und des Werkzeugelementträgers in distaler Richtung außer Eingriff gebracht werden können, um dann die Sperrelemente von der Sperrstellung in die Freigabestellung zu überführen.

Günstig ist es, wenn das Schiebeschaftinstrument eine Anschlageinrichtung umfasst zum Begrenzen einer Bewegung des Schiebeschafts in proximaler Richtung und in distaler Richtung. So kann beispielsweise eine Beschädigung von Implantaten, beispielsweise Aneurysmenclips, die mit dem Schiebeschaftinstrument gehandhabt werden sollen, einfach und sicher vermieden werden. Die durch die Anschlageinrichtung definierten maximalen Verschiebestellungen des Schiebeschafts und des Werkzeugelementträgers relativ zueinander definieren dann auch extreme Stellungen des mindestens einen Werkzeugelements am distalen Ende des Werkzeugelementträgers.

Vorteilhaft ist es, wenn die Anschlageinrichtung einen proximalen Anschlag und einen distalen Anschlag umfasst und wenn ein maximaler Verschiebeweg des Schiebeschafts relativ zum Werkzeugelementträger definiert wird durch einen Abstand von Wirkflächen des proximalen Anschlags und des distalen Anschlags voneinander. Beispielsweise können die Wirkflächen der Anschläge aufeinander zu weisen. Der Verschiebeweg wird dann durch den Abstand dieser Wirkflächen definiert. Optional können die Anschläge auch verstellbar ausgebildet sein. Dies ermöglicht es insbesondere, das Instrument werkseitig oder gegebenenfalls auch durch einen Nutzer zu justieren beziehungsweise nachzujustieren, um die Extremstellungen des Schiebeschafts und damit Extremstellungen des mindestens einen Werkzeugelements am distalen Ende des Werkzeugelementträgers in gewünschter Weise vorgeben zu können.

Vorzugsweise definieren der proximale Anschlag die Grundstellung und der distale Anschlag eine in distaler Richtung maximal ausgelenkte Stellung des Schiebeschafts. So kann ein Einsatz des Schiebeschaftinstruments in gewünschter Weise durch die Anordnung und Positionierung der Anschläge vorgegeben werden.

Günstig ist es, wenn die Sperreinrichtung eine Bewegung des Schiebeschafts relativ zum Werkzeugelementträger in mindestens einer Vorschubstellung zwischen der Grundstellung und der in distaler Richtung maximal ausgelenkten Stellung blockiert. So ist es insbesondere möglich, mit der Sperreinrichtung das Schiebeschaftinstrument in einer definierten Auslenkstellung des mindestens einen Werkzeugelements zu sperren. Beispielsweise kann es sich dabei um eine teilweise geöffnete oder teilweise geschlossene Stellung von miteinander zusammenwirkenden, von zwei oder mehr Werkzeugelementen definierenden Maulteilen des Schiebeschaftinstruments handeln.

Um die Reinigbarkeit des Schiebeschaftinstruments zu verbessern, ist es vorteilhaft, wenn der Werkzeugelementträger mit der Betätigungseinrichtung in einer Arbeitsstellung gekoppelt und in einer Reinigungsstellung von der Betätigungseinrichtung getrennt ist.

Die Reinigbarkeit des Schiebeschaftinstruments lässt sich zudem weiter verbessern dadurch, dass der Schiebeschaft mit der Betätigungseinrichtung in der Arbeitsstellung gekoppelt und in der Reinigungsstellung von der Betätigungseinrichtung getrennt ist.

Günstig ist es, wenn Betätigungseinrichtung einen Kopplungsteil umfasst, wenn die zwei Betätigungselemente am Kopplungsteil bewegbar angeordnet sind und wenn der Werkzeugelementträger in der Arbeitsstellung unbeweglich am Kopplungsteil gehalten ist. Eine solche Ausgestaltung kann insbesondere sicherstellen, dass sich ein distales Ende des Werkzeugelementträgers relativ zum Kopplungsteil und damit zur Betätigungseinrichtung nicht in der Verschieberichtung bewegt. So kann ein Operateur das Schiebeschaftinstrument in gewohnter Weise führen und halten. Insbesondere kann das mindestens eine erste Sperrelement einstückig, beispielsweise monolithisch, am Kopplungsteil angeordnet oder ausgebildet werden. So kann eine Anzahl von lösbar verbindbaren Teilen des Schiebeschaftinstruments minimiert werden.

Vorteilhaft ist es, wenn der Kopplungsteil ein in der Verschieberichtung verschiebbar gelagertes Mitnehmerglied umfasst und wenn das Mitnehmerglied über eine Lenkeranordnung mit den zwei Betätigungselementen gekoppelt ist zum Bewegen des Mitnehmerglieds in der Verschieberichtung infolge einer Bewegung der zwei Betätigungselemente aufeinander zu oder voneinander weg.

Beispielsweise kann bei einem zerlegbaren Schiebeschaftinstrument der Schiebeschaft mit seinem proximalen Ende mit dem Mitnehmerglied kraft- und/oder formschlüssig in Eingriff gebracht werden, so dass er bei einer Bewegung des Mitnehmerglieds entsprechend mitbewegt wird.

Vorteilhaft ist es, wenn ein proximales Ende des Schiebeschafts in der Arbeitsstellung mit dem Mitnehmerglied gekoppelt ist. So kann insbesondere der Schiebeschaft von der Betätigungseinrichtung gelöst werden, indem das proximale Ende desselben vom Mitnehmerglied entkoppelt wird, so dass der Schiebeschaft die Reinigungsstellung einnimmt, in der er von der Betätigungseinrichtung getrennt ist.

Vorteilhaft ist es, wenn das mindestens eine Rückstellelement einerseits am Kopplungsteil und andererseits am mindestens einen ersten Sperrelement angreift. Insbesondere kann es am Hebelarm des mindestens einen Sperrelements angreifen. Durch diese Anordnung des mindestens einen Rückstellelements der Rückstelleinrichtung kann insbesondere sichergestellt werden, dass die Sperreinrichtung automatisch in die Freigabestellung überführt wird, wenn die Sicherungseinrichtung entsichert ist.

Auf besonders einfache Weise lässt sich das Schiebeschaftinstrument in einer Vorschubstellung sichern, wenn das mindestens eine erste Sperrelement in Form einer in der Sperrstellung am Schiebeschaft angreifenden Sperrklinke ausgebildet ist. Die Sperrklinke kann insbesondere federnd vorgespannt sein, damit sie automatisch in die Freigabestellung überführt wird. Insbesondere kann die Sperrklinke die Rastnase umfassen, die mit einer Hinterschneidung am Schiebeschaft, insbesondere am mindestens einen zweiten Sperrelement, zusammenwirkt.

Um beliebige Schaftformen realisieren zu können, ist es vorteilhaft, wenn die Verschieberichtung geradlinig oder gekrümmt verläuft. Es können dann insbesondere geradlinige Schiebeschaftinstrument realisiert werden, bei denen die Verschieberichtung definiert wird durch eine Längsachse des Werkzeugelementträgers beziehungsweise eine Längsachse des Schiebeschafts. Alternativ kann eine gekrümmte Schieberichtung definiert werden durch einen gekrümmten Werkzeugelementträger und einen korrespondierend gekrümmten Schiebeschaft.

Um ein besonders kompaktes Schiebeschaftinstrument ausbilden zu können, ist es vorteilhaft, wenn das mindestens eine Werkzeugelement und der Werkzeugelementträger einstückig ausgebildet sind. So kann insbesondere auf in der Herstellung aufwendige bewegliche Lagerungen des mindestens einen Werkzeugelements am Werkzeugelementträger verzichtet werden. Chirurgische Clips, insbesondere Aneurysmenclips, lassen sich mit dem Schiebeschaftinstrument einfach und sicher handhaben, wenn das Schiebeschaftinstrument in Form einer Clipanlegezange ausgebildet ist.

Die vorliegende Erfindung wird im Hauptanspruch 1 definiert. Die weiteren Ausführungsformen werden in den Nebenansprüchen definiert.

Die nachfolgende Beschreibung bevorzugter Ausführungsformen der Erfindung dient im Zusammenhang mit den Zeichnungen der näheren Erläuterung. Es zeigen:
- Figur 1:: eine schematische perspektivische Gesamtansicht eines Ausführungsbeispiels eines Schiebeschaftinstruments;
- Figur 2:: eine vergrößerte Teilansicht des Bereichs A aus Figur 1 mit am Schiebeschaftinstrument gehaltenem chirurgischem Clip;
- Figur 3:: eine Seitenansicht des Schiebeschaftinstruments aus Figur 1;
- Figur 4:: eine Ansicht des Schiebeschaftinstruments aus Figur 3 in Richtung des Pfeils B;
- Figur 5:: eine Teilansicht des Schiebeschaftinstruments aus Figur 3 in Richtung des Pfeils C;
- Figur 6:: eine Ansicht ähnlich Figur 5 mit etwas in distaler Richtung relativ zum Werkzeugelementträger bewegtem Schiebeschaft;
- Figur 7:: eine ausschnittsweise Längsschnittansicht des Schiebeschaftinstruments mit einem aus der Grundstellung ausgelenkten Schiebeschaft vor Erreichen der Sperrstellung;
- Figur 8:: eine Schnittansicht des Schiebeschaftinstruments aus Figur 6 längs Linie 8-8;
- Figur 9:: eine vergrößerte Teilansicht der Anordnung aus Figur 9 bei ineinandergreifenden Sperrelementen;
- Figur 10:: eine vergrößerte Ansicht eines Teils der Anordnung aus Figur 8;
- Figur 11:: eine Ansicht ähnlich Figur 10 eines weiteren Ausführungsbeispiels eines Schiebeschaftinstruments;
- Figur 12:: eine Ansicht ähnlich Figur 10 eines weiteren Ausführungsbeispiels eines Schiebeschaftinstruments;
- Figur 13:: eine Ansicht ähnlich Figur 10 eines weiteren Ausführungsbeispiels eines Schiebeschaftinstruments; und
- Figur 14:: eine Ansicht ähnlich Figur 10 eines weiteren Ausführungsbeispiels eines Schiebeschaftinstruments.

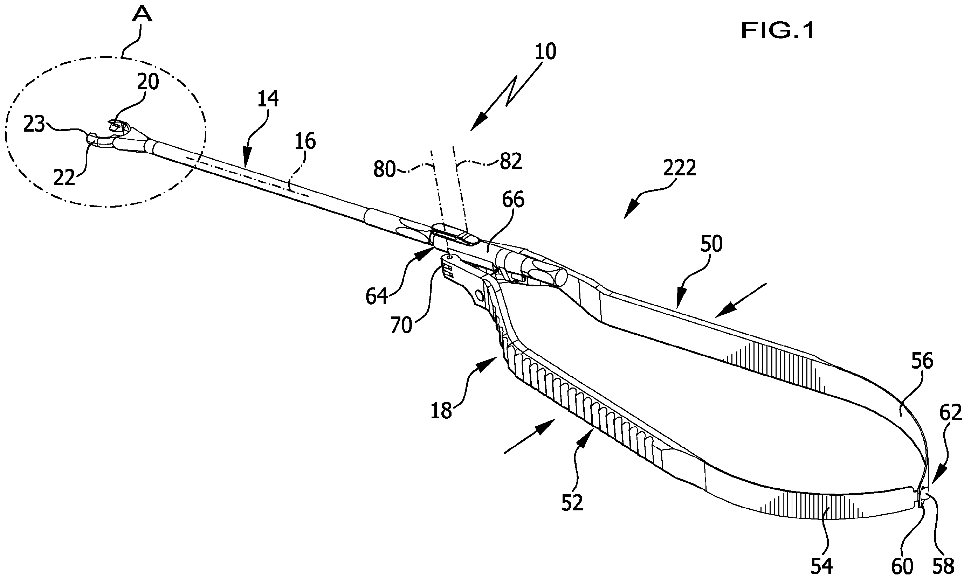

Ein erstes Ausführungsbespiel eines medizinischen Schiebeschaftinstruments ist schematisch in Figur 1 dargestellt und insgesamt mit dem Bezugszeichen 10 bezeichnet. Es umfasst einen Werkzeugelementträger 12, welcher von einem rohrförmigen Schiebeschaft 14 umgeben ist.

Der Schiebeschaft 14 definiert eine Verschieberichtung 16, und zwar durch seine Längsachse 148. Die Verschieberichtung 16 verläuft somit bei dem in Figur 1 dargestellten Ausführungsbeispiel geradlinig. Alternativ kann bei einem nicht dargestellten Ausführungsbeispiel die Verschieberichtung 16 auch gekrümmt verlaufen.

Das Schiebeschaftinstrument 10 umfasst ferner eine Betätigungseinrichtung 18 zum Bewegen des Schiebeschafts 14 relativ zum Werkzeugelementträger 12 sowohl in distaler als auch in proximaler Richtung.

Am distalen Ende des Werkzeugelementträgers 12 sind bei dem dargestellten Ausführungsbeispiel zwei Werkzeugelemente 20 und 22 angeordnet. Sie sind einstückig, nämlich monolithisch, mit dem Werkzeugelementträger 12 ausgebildet. Sie sind in Form langgestreckter Arme ausgebildet, die ausgehend von einem distalen Ende 23 des Werkzeugelementträgers 12 durch einen Schlitz 24 voneinander getrennt sind.

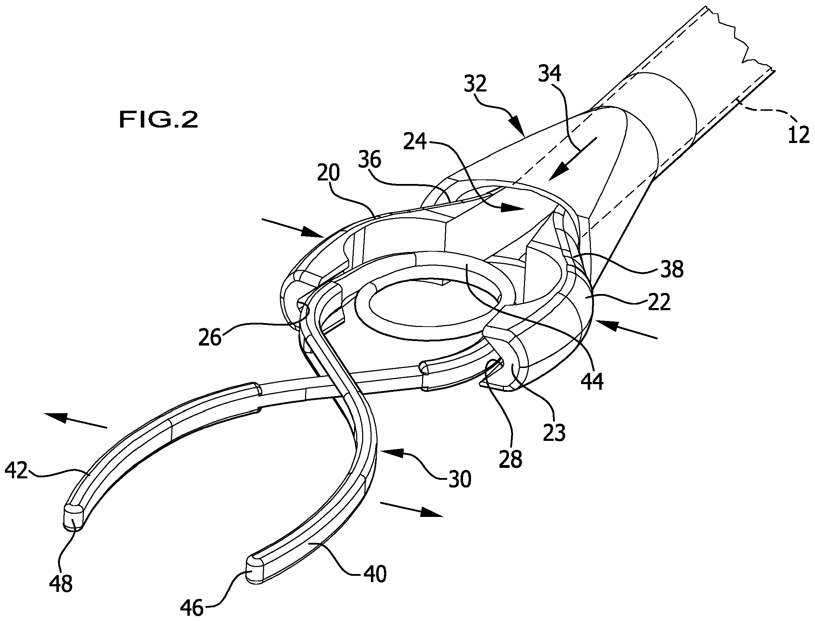

Die beiden Werkzeugelemente 20, 22 sind in einer Grundstellung, in welcher keine äußeren Kräfte auf sie einwirken, wie in den Figuren 1 und 2 schematisch dargestellt, auseinandergespreizt.

Die Werkzeugelemente 20 und 22 weisen aufeinander zu weisende Halteflächen 26 und 28 auf, die etwas vertieft ausgebildet sind, um beispielsweise einen in Figur 2 schematisch dargestellten chirurgischen Clip 30 aufzunehmen.

Zum Schließen der Werkzeugelemente 20 und 22 dient ein sich in distaler Richtung trichterförmig erweiternder Endabschnitt 32 des Schiebeschafts 14. Wird der Schiebeschaft 14 in distaler Richtung bewegt, wie dies durch den Pfeil 34 in Figur 2 angedeutet ist, gleitet der Endabschnitt 32 an voneinander weg weisenden Außenflächen 36 und 38 der Werkzeugelemente 20 und 22 auf, sodass diese aufeinander zu bewegt werden. Durch diese Bewegung der Werkzeugelemente 20 und 22 wird der zwischen diesen aufgenommene Clip 30 nicht nur klemmend gehalten, sondern auch mit zunehmender Annäherung der Werkzeugelemente 20 und 22 aneinander sukzessive geöffnet. Diese Funktionsweise ergibt sich beim Clip 30 dadurch, dass sich dessen Klemmarme 40 und 42, die proximalseitig mit einem schraubenförmigen Federelement verbunden sind, kreuzen. Mit anderen Worten ist der Clip 30 derart ausgestaltet, dass er ohne auf ihn einwirkende äußere Kräfte eine Schließstellung einnimmt, in welcher die Klemmarme 40 und 42 mit ihren freien Enden 46 und 48 eine maximal angenäherte Stellung einnehmen.

In der beschriebenen Weise sind die Werkzeugelemente 20 und 22 mit dem einen distalen Endbereich bildenden Endabschnitt 32 des Schiebeschafts 14 zusammenwirkend angeordnet und ausgebildet derart, dass infolge einer Bewegung des Schiebeschafts in distaler Richtung die Werkzeugelemente 20 und 22 bewegt werden, und zwar bei dem dargestellten Ausführungsbeispiel aufeinander zu.

Die Betätigungseinrichtung 18 umfasst beim Schiebeschaftinstrument 10 zwei relativ zueinander bewegbare, nämlich verschwenkbare, Betätigungselemente 50 und 52. Proximale Endabschnitte der in Form von Branchen ausgebildeten Betätigungselemente 50 und 52 sind in Form von blattfederartigen Vorspannelementen 54 und 56 ausgebildet. Freie Enden 58 und 60 derselben sind miteinander beweglich in Eingriff stehend. Die Vorspannelemente 54 und 56 bilden eine Vorspanneinrichtung 62 zum Ausüben einer in proximaler Richtung wirkenden vorspannenden Kraft auf den Schiebeschaft 14.

Die Vorspannelemente 54 und 56 sind aufeinander zuweisend gekrümmt und halten in einer Grundstellung die Betätigungselemente 50 und 52 in einer maximal voneinander entfernten Stellung. Werden die Betätigungselemente 50 und 52 wie durch die Pfeile in Figur 1 schematisch dargestellt aufeinander zu bewegt, erfolgt dies entgegen der Wirkung der Vorspanneinrichtung 62, also entgegen der Wirkung der Vorspannelemente 54 und 56.

Die Betätigungseinrichtung 18 umfasst einen Kopplungsabschnitt 64, auch als Kopplungsteil bezeichnet, mit einer Hülse 66, von welcher seitlich, quer zur Verschieberichtung 16 und voneinander weg weisend Lagerbacken 68 und 70 abstehen, an denen in distaler Richtung weisende Enden 72 und 74 der Betätigungselemente 50 und 52 mittels Lagerstiften 76 und 78 verschwenkbar gelagert sind. Die beiden Lagerstifte 76 und 78 definieren jeweils eine Schwenkachse 80 beziehungsweise 82, welche quer, bei dem in den Figuren dargestellten Ausführungsbeispiel senkrecht, zur Verschieberichtung 16 verläuft.

In der Hülse 66 ist ein in der Verschieberichtung 16 verschiebbar gelagertes Mitnehmerglied 84 angeordnet. Das Mitnehmerglied 84 umfasst einen in der Hülse 16 verschiebbar gelagerten Hülsenabschnitt 86, von dem ein Kupplungszapfen aus einem in Form einer Durchbrechung, die sich in der Verschieberichtung 16 erstreckt, ausgebildeten Fenster 90 vorsteht.

Am Kupplungszapfen 88 sind um eine gemeinsame Schwenkachse 92, die parallel zu den Schwenkachsen 80 und 82 verläuft, zwei Lenker 94 und 96 einer Lenkeranordnung 98 verschwenkbar gelagert. Jeweils die anderen Enden der Lenker 94 und 96 sind jeweils proximalseitig der Lagerbacken 68 und 70 an Lagerbacken 100 beziehungsweise 102, die von den Betätigungselementen 50 und 52 aufeinander zuweisend abstehend angeordnet sind, mittels Lagerstiften 104 beziehungsweise 106 verschwenkbar gelagert. Die Lagerstifte 104 und 106 definieren mit ihren Längsachsen Schwenkachsen, die parallel zur Schwenkachse 92 verlaufen.

Durch die Kopplung der Betätigungselemente 50 und 52 in der beschriebenen Weise mittels der Lenkeranordnung 98 am Kupplungszapfen 88 des Mitnehmerglieds 84, wird das Mitnehmerglied 84 bei einer Bewegung der Betätigungselemente 50 und 52 aufeinander zu in distaler Richtung bewegt, bei einer Bewegung der Betätigungselemente 50 und 52 voneinander weg in proximaler Richtung.

Das Schiebeschaftinstrument 10 umfasst ferner eine Anschlageinrichtung 108 zum Begrenzen einer Bewegung des Schiebeschafts 14 in proximaler Richtung und in distaler Richtung. Hierfür umfasst die Anschlageinrichtung 108 einen proximalen Anschlag 110 und einen distalen Anschlag 112. Die Anschläge 110 und 112 sind gebildet durch mit einem Außengewinde versehene Madenschrauben 114 beziehungsweise 116, die in mit Innengewinden 122 beziehungsweise 124 versehenen, sich parallel zur Verschieberichtung 16 erstreckenden Bohrungen 126 und 128 an von der Hülse 66 parallel zum Kupplungszapfen 88 abstehenden Anschlagvorsprüngen 118 und 120 eingeschraubt sind.

Ein maximaler Verschiebeweg 130 des Mitnehmerglieds 84 relativ zur Hülse 66 wird definiert durch einen Abstand 132 von Wirkflächen 134 und 136 des proximalen Anschlags 110 und des distalen Anschlags 112 voneinander. Bei dem in den Figuren dargestellten Ausführungsbeispiel weisen die Wirkflächen 134 und 136 aufeinander zu und dienen zur Begrenzung einer Bewegung des Kupplungszapfens 88 in proximaler beziehungsweise distaler Richtung.

Der Schiebeschaft 14 ist in einer Grundstellung des Schiebeschaftinstruments 10 durch die Vorspanneinrichtung 62 maximal weit in proximaler Richtung verschoben. Der proximale Anschlag 110 definiert die Grundstellung. Der distale Anschlag 112 definiert eine in distaler Richtung maximal ausgelenkte Stellung des Schiebeschafts 14.

Der Schiebeschaft 14 ist in einer Arbeitsstellung mit der Betätigungseinrichtung 18 gekoppelt. Um dies zu ermöglichen, ist ein proximaler Endabschnitt 138 des Schiebeschafts 14 mit zwei zueinander senkrechten Schlitzen versehen, wodurch vier sich parallel zur Verschieberichtung 16 erstreckende Federarme 140 ausgebildet sind.

An den Federarmen ist in radialer Richtung nach außen weg weisend eine ringförmige Vertiefung 142 ausgebildet, in die der Hülsenabschnitt 86 in der Arbeitsstellung eingreift. Eine Außenfläche eines sich an die Vertiefung 142, die in Form einer Ringnut ausgebildet ist, anschließenden Ringvorsprungs 144 weist in proximaler Richtung geneigte Aufgleitflächen 146 auf.

Wird der Schiebeschaft 14 aus einer Reinigungsstellung, in welcher er vollständig von der Betätigungseinrichtung 18 getrennt ist, mit den Federarmen 140 in proximaler Richtung in den Hülsenabschnitt 86 des Mitnehmerglieds 84 eingeführt, gleiten die Aufgleitflächen 146 am Hülsenabschnitt 86 auf, so dass die freien Enden der Federarme 140, die in proximaler Richtung weisen, in Richtung auf die Längsachse 148 des Schiebeschafts 14 verschwenkt werden.

Die Federarme 140 schwenken wieder von der Längsachse 148 weg nach au-ßen aus, sobald der Hülsenabschnitt 86 vollständig in der ringnutförmigen Vertiefung 142 aufgenommen ist. Der Schiebeschaft 14 ist so mit dem Mitnehmerglied 84 der Betätigungseinrichtung 18 in der Arbeitsstellung gekoppelt.

Der Werkzeugelementträger 12 ist ausgehend von einem proximalen Ende 150 mit einem Schlitz 152 versehen. So sind, ähnlich wie am Schiebeschaft 14, zwei Federarme 154 am Werkzeugelementträger 12 ausgebildet, die sich parallel zur Längsachse 148 in proximaler Richtung weisend erstrecken. Etwas vom Ende 150 beabstandet ist an den Federarmen 154 eine Ringnut 156 ausgebildet, in welche ein in Richtung auf die Längsachse 148 vorspringender Ringvorsprung 158 eingreift, wenn der Werkzeugelementträger 12 mit dem Kopplungsabschnitt 64 gekoppelt ist.

Ein Außendurchmesser des Werkzeugelementträgers 12 ist an einen Innendurchmesser des Schiebeschafts 14 angepasst, so dass nach dem Koppeln des Schiebeschafts 14 mit dem Mitnehmerglied 84 in der oben beschriebenen Weise der Werkzeugelementträger 12 von distaler Richtung her kommend mit dem Ende 150 voran in proximaler Richtung durch den Schiebeschaft 14 hindurchgeschoben werden kann. Das Ende 150 gleitet am Ringvorsprung 150 auf, so dass die Federarme 154 aufeinander zu verschwenkt werden, bis der Ringvorsprung 158 in die Ringnut 156 eintauchen kann.

Um den Werkzeugelementträger 12 und den Schiebeschaft 14 in der beschriebenen Arbeitsstellung zu sichern, ist am Kopplungsabschnitt 64 ein Verriegelungsstift 160 verschiebbar gelagert. Der Verriegelungsstift 160 steht von einem Schubglied 162 in distaler Richtung weisend ab. Das Schubglied 162 ist durch ein vorspannendes Element 164 in Form einer Schraubenfeder in distaler Richtung vorgespannt.

Der Verriegelungsstift 160 ist so bemessen, dass er in der Arbeitsstellung zwischen die Federarme 154 des Werkzeugelementträgers 12 in den Schlitz 152 eintaucht, wodurch eine Bewegung der Federarme 154 aufeinander zu verhindert wird. Durch diese Positionierung der Federarme 154 in der Arbeitsstellung wird gleichzeitig durch die Federarme 154 ein Einschwenken der Federarme 140 des Schiebeschafts 14 in Richtung auf die Längsachse 148 hin verhindert. Der Verriegelungsstift 160 verriegelt somit direkt den Werkzeugelementträger 12 in der Arbeitsstellung am Kopplungsabschnitt 64 der Betätigungseinrichtung 18 und indirekt den Schiebeschaft 14 am Mitnehmerglied 84 der Betätigungseinrichtung 18.

Zum Zerlegen des Schiebeschaftinstruments 10 wird das Schubglied 162, dessen proximales Ende von einer Kappe 166 umgeben ist, in proximaler Richtung entgegen der Wirkung des vorspannenden Elements 164 parallel zur Längsachse 148 verschoben. Der Verriegelungsstift 160 gibt dann die Federarme 154 frei, so dass der Werkzeugelementträger 12 in distaler Richtung aus dem Schiebeschaft 14 herausgezogen werden kann.

Ist der Werkzeugelementträger 12 aus dem Schiebeschaft 14 herausgezogen, können auch die Federarme 140 in Richtung auf die Längsachse 140 verschwenkt werden, indem der Schiebeschaft 14 in distaler Richtung gezogen wird.

Durch die Kopplung des Werkzeugelementträgers 12 in der beschriebenen Weise mit der Betätigungseinrichtung 18 ist der Werkzeugelementträger 12 in der Arbeitsstellung unbeweglich am Kopplungsabschnitt 64 gehalten.

Das Schiebeschaftinstrument 10 umfasst ferner eine Sperreinrichtung 168 zum Blockieren einer Bewegung des Schiebeschafts 14 und des Werkzeugelementträgers 12 in einer Vorschubstellung, in welcher der Schiebeschaft 14 gegenüber der oben erwähnten Grundstellung, in welcher der Schiebeschaft 14 relativ zum Werkzeugelementträger 12 maximal weit in proximaler Richtung verschoben ist, in distaler Richtung verschoben ist.

Die Sperreinrichtung 168 definiert eine Freigabestellung, in welcher der Schiebeschaft 14 und der Werkzeugelementträger 12 parallel zur Längsachse 148 beziehungsweise parallel zur Verschieberichtung 16 verschoben werden können. Ferner definiert die Sperreinrichtung 168 eine Sperrstellung, in welcher eine Bewegung des Schiebeschafts 14 relativ zum Werkzeugelementträger 12 in zumindest proximaler Richtung blockiert ist.

Die Sperreinrichtung 168 umfasst ein erstes Sperrelement 170 und ein zweites Sperrelement 172.

Die Sperrelemente 170 und 172 greifen in der Sperrstellung kraft- und/oder formschlüssig ineinander ein. In der Freigabestellung stehen sie außer Eingriff.

Das zweite Sperrelement 172 ist am Schiebeschaft 14 angeordnet oder ausgebildet. Das erste Sperrelement 170 ist von der Freigabestellung durch eine Bewegung in Richtung auf den Schiebeschaft 14 hin in die Sperrstellung überführbar.

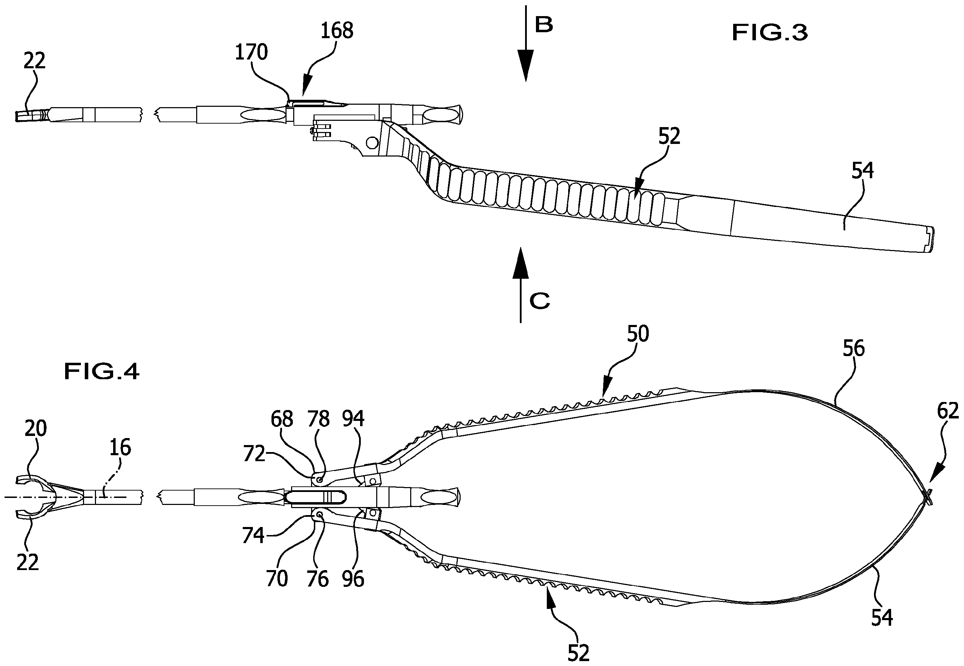

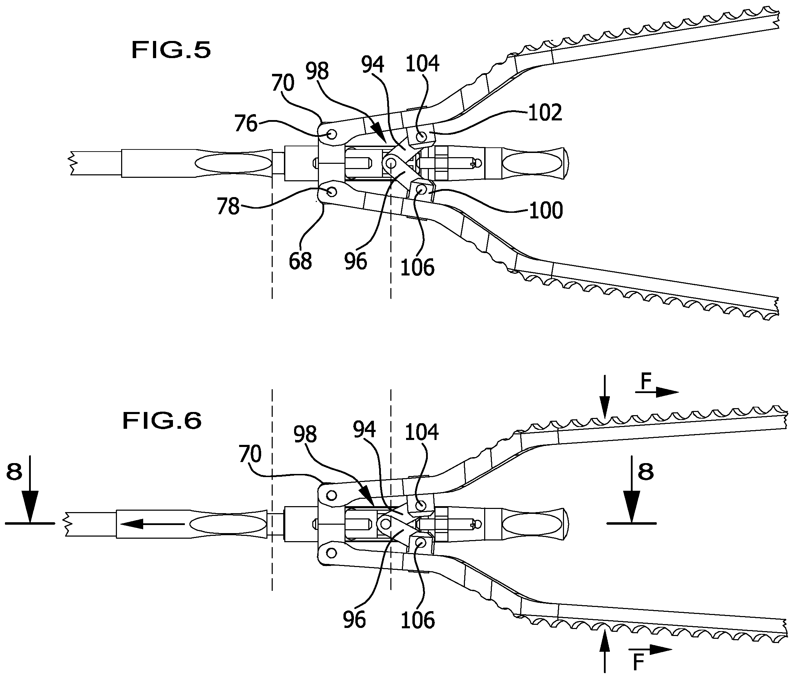

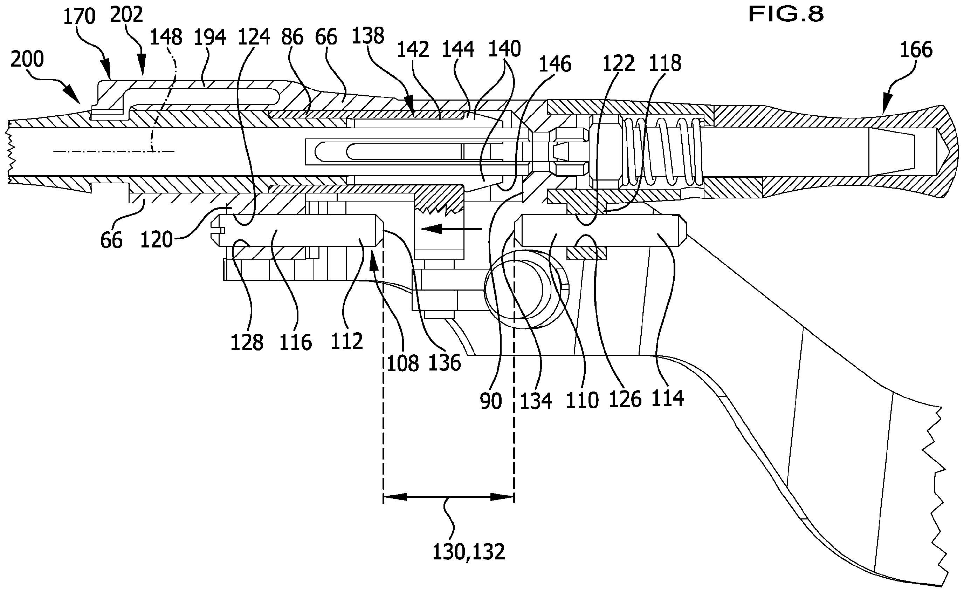

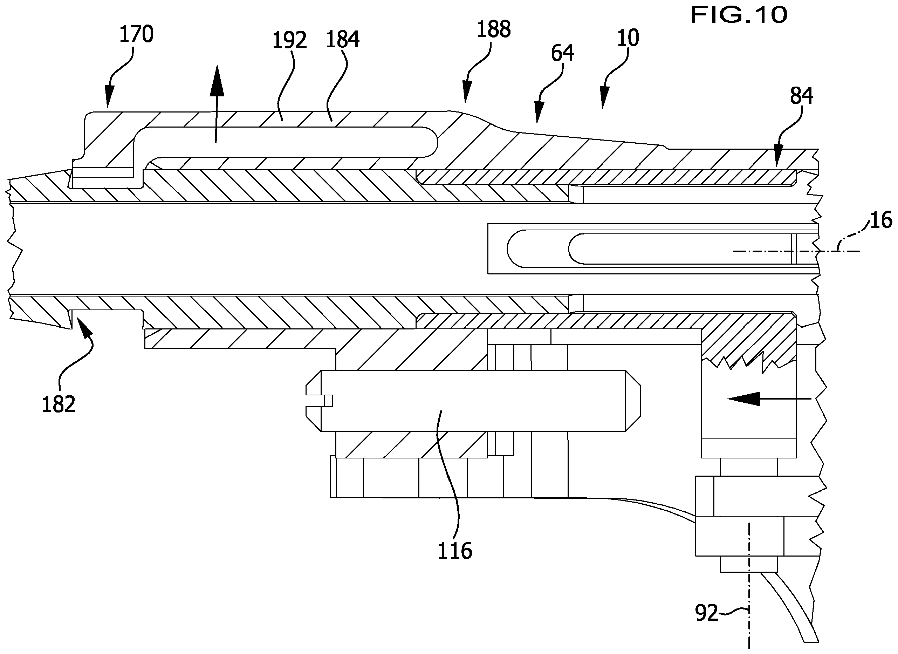

Das zweite Sperrelement 172 und der Schiebeschaft 14 sind einstückig, nämlich monolithisch, ausgebildet. Bei dem in den Figuren 1 bis 10 dargestellten Ausführungsbeispiel des Schiebeschaftinstruments 10 ist das zweite Sperrelement 172 in Form einer vom Schiebeschaft 14 weg weisend geöffneten Rückhaltenut 174 ausgebildet. Diese umgibt den Schiebeschaft 14 ringförmig und ist in sich geschlossen. Die Rückhaltenut 174 ist somit in Form einer Ringnut ausgebildet.

Das zweite Sperrelement 172 umfasst eine in proximaler Richtung weisende Anschlagfläche 176. Die Anschlagfläche 176 bildet eine Seitenwand der Rückhaltenut 174.

Die Anschlagfläche 176 ist etwas in Richtung auf den Schiebeschaft 14 hin weisend geneigt. Ein Neigungswinkel 178 zwischen der Anschlagfläche 176 und der Verschieberichtung 16 liegt in einem Bereich von etwa 88° bis etwa 60°.

Die Anschlagfläche 176 definiert zusammen mit einer von der Längsachse 148 in radialer Richtung weg weisenden Ringfläche 180 der Rückhaltenut 174 eine Hinterschneidung 182, die am zweiten Sperrelement 172 ausgebildet ist.

Das erste Sperrelement 170 ist am Schiebeschaftinstrument 10 derart angeordnet, dass es relativ zum Werkzeugelementträger 12 in der Verschieberichtung 16 unbeweglich oder im Wesentlichen unbeweglich angeordnet oder ausgebildet ist. Dies wird dadurch erreicht, dass das erste Sperrelement am Kopplungsabschnitt 84 angeordnet beziehungsweise ausgebildet ist, relativ zu dem Werkzeugelementträger 12 in der Arbeitsstellung in axialer Richtung, also parallel zur Verschieberichtung 16, unbeweglich gehalten ist.

Das erste Sperrelement 170 umfasst einen Hebelarm 184, welcher sich in distaler Richtung weisend parallel zur Verschieberichtung 16 erstreckt. Der Hebelarm 184 ist proximalseitig am Schiebeschaftinstrument 10 festgelegt, und zwar dadurch, dass er einstückig mit dem Kopplungsabschnitt 64 ausgebildet ist.

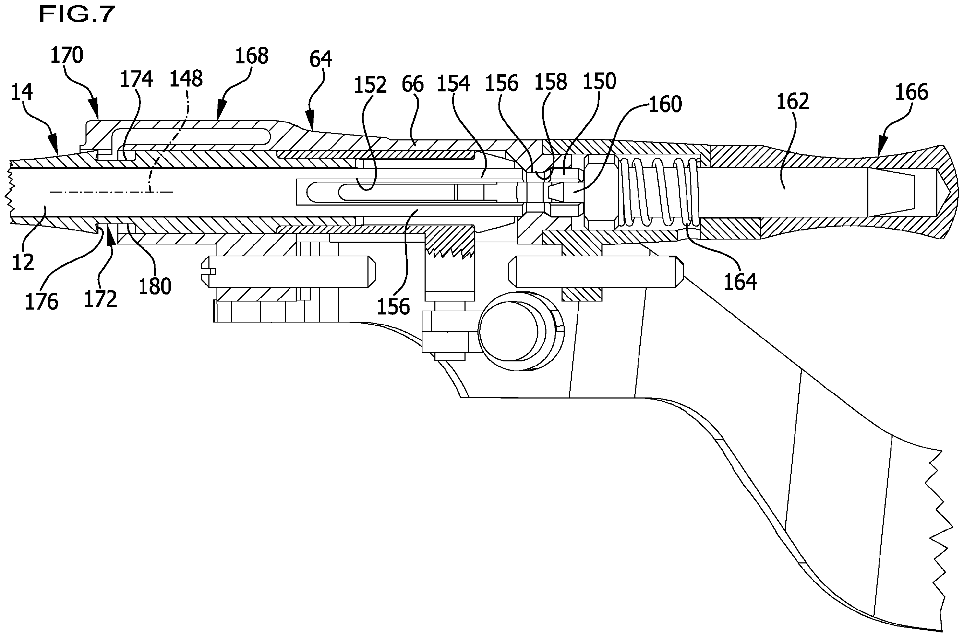

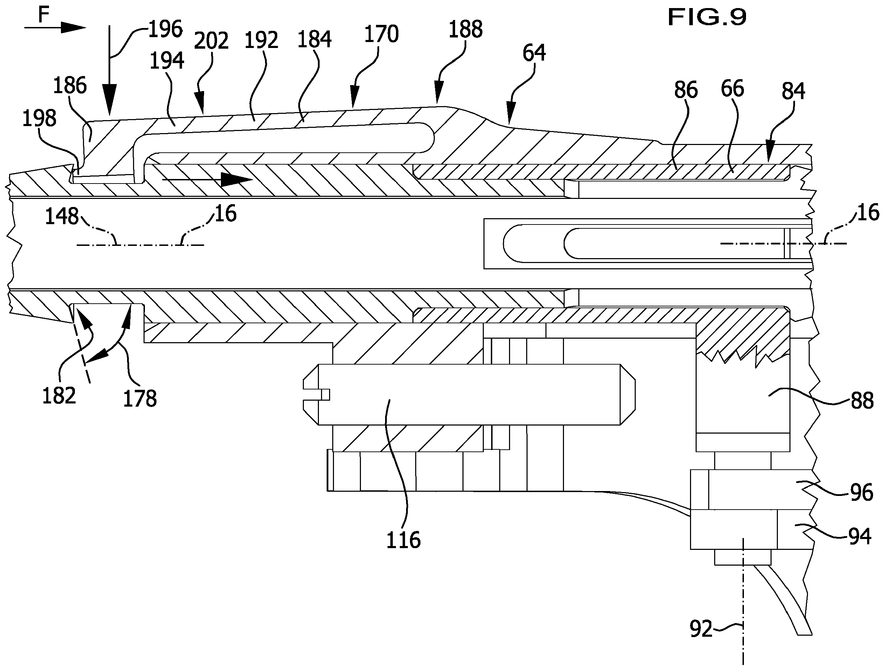

Das erste Sperrelement 170 umfasst ferner einen in Richtung auf den Schiebeschaft 14 weisenden Sperrvorsprung 186, welcher in der Freigabestellung mit dem zweiten Sperrelement 172 außer Eingriff steht. Die Freigabestellung ist beispielhaft in Figur 7 dargestellt. In der Sperrstellung steht der Sperrvorsprung 186, wie schematisch in Figur 9 dargestellt, mit dem zweiten Sperrelement 172 in Eingriff.

Die Sperreinrichtung 168 umfasst ferner eine Rückstelleinrichtung 188. Die Rückstelleinrichtung 188 ist derart angeordnet und ausgebildet, dass das erste Sperrelement 170 entgegen der Wirkung der Rückstelleinrichtung 188 von der Freigabestellung in die Sperrstellung bewegbar ist.

Die Rückstelleinrichtung 188 umfasst ein Rückstellelement 190, welches mit dem ersten Sperrelement 170 zusammenwirkend angeordnet oder ausgebildet ist.

Bei dem in den Figuren 1 bis 10 dargestellten Ausführungsbeispiel des Schiebeschaftinstruments 10 ist das Rückstellelement 190 in Form eines Federelements 192 ausgebildet, und zwar in Form einer Blattfeder 194. Zudem umfasst der Hebelarm 184 das Rückstellelement 190 beziehungsweise bildet dieses.

Wie schematisch in Figur 9 dargestellt kann durch Ausüben einer Druckkraft, symbolisiert durch den Pfeil 196, auf ein freies Ende des Hebelarms 184 das erste Sperrelement 170 entgegen der Wirkung des Rückstellelements 190 mit dem Sperrvorsprung 186 in Richtung auf die Längsachse 148 verschwenkt werden.

Das erste Sperrelement 170 umfasst ferner eine Rastnase 198. Die Rastnase 198 greift in der Sperrstellung formschlüssig oder im Wesentlichen formschlüssig in die Hinterschneidung 182 am zweiten Sperrelement 172 ein, wie dies schematisch in Figur 9 dargestellt ist. Die Rastnase 198 ist in distaler Richtung weisend angeordnet oder ausgebildet, und zwar bei dem in den Figuren dargestellten Ausführungsbeispiel am Sperrvorsprung 186. Dieser ist wie beschrieben an einem freien Ende des Hebelarms 184 angeordnet oder ausgebildet.

Das Schiebeschaftinstrument 10 umfasst ferner eine Sicherungseinrichtung 200 zum Sichern der Sperreinrichtung 168 in der Sperrstellung.

Die Sicherungseinrichtung 200 umfasst das erste Sperrelement 170 und das zweite Sperrelement 172, die derart angeordnet oder ausgebildet sind, dass die Sperreinrichtung 168 nur durch eine Bewegung des Schiebeschafts 14 in distaler Richtung entsichert werden kann. Dies wird bei dem in den Figuren 1 bis 10 dargestellten Ausführungsbeispiel des Schiebeschaftinstruments 10 dadurch erreicht, dass die Sicherungseinrichtung 200 die Hinterschneidung 182 und die Rastnase 198 umfasst.

Stehen die Hinterschneidung 182 und die Rastnase 198 in Eingriff, ist das Schiebeschaftinstrument 10 in der Sperrstellung gesichert.

Zum Lösen der Sperrstellung muss der Schiebeschaft 14 etwas in distaler Richtung verschoben werden. Dies wird dadurch erreicht, dass die Betätigungselemente 50 und 52 etwas aufeinander zu verschwenkt werden, wodurch im Kopplungsabschnitt 64 das Mitnehmerglied 84 mit dem daran gekoppelten Schiebeschaft 14 etwas in distaler Richtung verschoben wird. Die Rastnase 198 und die Hinterschneidung 182 gelangen dabei außer Eingriff und das Rückstellelement 190 bewegt den Sperrvorsprung 186 aus der Rückhaltenut 174 heraus. Das Schiebeschaftinstrument 10 nimmt nun die Freigabestellung ein. Der Schiebeschaft 14 kann relativ zum Werkzeugelementträger 12 im Rahmen des vorgegebenen Verschiebewegs 130 in der Verschieberichtung 16 in distaler und proximaler Richtung verschoben werden.

Zum Überführen des Schiebeschaftinstruments 10 von der Freigabestellung in die Sperrstellung muss zunächst der Schiebeschaft 14 so weit in distaler Richtung vorgeschoben werden, dass der Sperrvorsprung 186 mit der Rastnase 198 in die Rückhaltenut 174 eintauchen kann. Das Eingreifen des Sperrvorsprungs 186 erfolgt durch Ausüben einer Druckkraft auf das freie Ende des Hebelarms 184.

Werden die Betätigungselemente 50 und 52 bei vorgeschobenem Sperrvorsprung 186 nicht weiter zusammengedrückt, bewegt die Vorspanneinrichtung 62 durch Ausüben einer in proximaler Richtung wirkenden vorspannenden Kraft auf die Betätigungselemente 50 und 52 den mit der Betätigungseinrichtung 18 beweglich gekoppelten Kopplungsabschnitt 64 etwas in proximaler Richtung, und zwar so weit, bis die Rastnase 198 in die Hinterschneidung 182 eingreift. Das Schiebeschaftinstrument 10 ist nun in einer Vorschubstellung des Schiebeschafts 14, die zwischen den Extremstellungen in proximaler und distaler Richtung definiert ist, gegen eine Bewegung des Schiebeschafts 14 in proximaler Richtung relativ zum Werkzeugelementträger 12 blockiert und gesichert.

Das beschriebene erste Sperrelement 170 ist bei dem in den Figuren 1 bis 10 dargestellten Ausführungsbeispiel des Schiebeschaftinstruments 10 in Form einer in der Sperrstellung direkt am Schiebeschaft 14 angreifenden Sperrklinke 202 ausgebildet.

Das im Zusammenhang mit den Figuren 1 bis 10 beschriebene Schiebeschaftinstrument 10 kann wie bereits erläutert eingesetzt werden, um einen Clip 30 in einer teilweise geöffneten Stellung zu halten und handzuhaben, insbesondere einem Operateur von einer ihm assistierenden Person anzureichen.

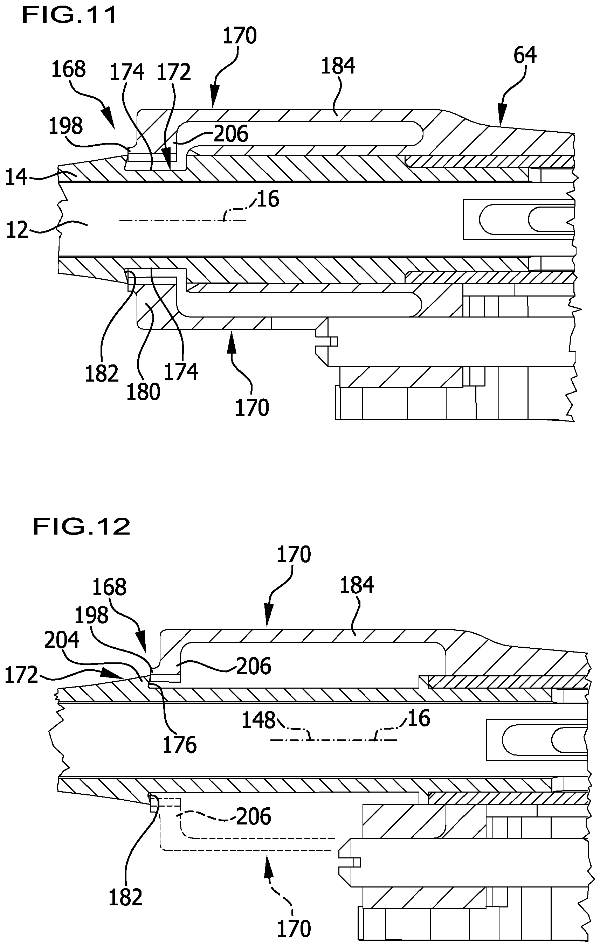

Der Grundaufbau des Schiebeschaftinstruments 10 kann bei weiteren Ausführungsbeispielen beibehalten werden. Varianten der Sperreinrichtung 168 des Schiebeschaftinstruments 10 werden nachfolgend in Verbindung mit den Figuren 11 bis 14 näher erläutert.

Das Ausführungsbeispiel der Sperreinrichtung 168, wie es schematisch in Figur 11 dargestellt ist, umfasst zwei erste Sperrelemente 170. Diese sind am Kopplungsabschnitt 64 einander gegenüberliegend angeordnet, sodass zum Blockieren einer Bewegung des Schiebeschafts 14 relativ zum Werkzeugelementträger 12 in proximaler Richtung nur einer der beiden Sperrvorsprünge 168 in die Rückhaltenut 174 eingeführt werden muss. Die Sperreinrichtung 168 mit zwei ersten Sperrelementen 170 auszubilden, ermöglicht insbesondere eine flexiblere Handhabung des Schiebeschaftinstruments 10.

Bei dem Ausführungsbeispiel, das in Figur 12 dargestellt ist, ist statt einer Rückhaltenut das zweite Sperrelement 172 in Form eines vom Schiebeschaft 14 in radialer Richtung weg weisenden Rückhaltevorsprungs 204 ausgebildet. Der Rückhaltevorsprung 204 umgibt den Schiebeschaft 14 ebenfalls ringförmig und ist in sich geschlossen. Der Rückhaltevorsprung 204 ist somit in Form eines Ringvorsprungs ausgebildet.

Der Ringvorsprung weist eine in proximaler Richtung weisende Anschlagfläche 176 auf, die wiederum bezogen auf die Verschieberichtung 16 beziehungsweise die Längsachse 148 geneigt ist und einen Neigungswinkel 178 definiert. Auf diese Weise wird wiederum eine Hinterschneidung 182 ausgebildet, in die eine Rastnase 198 am Sperrvorsprung 184 in der Sperrstellung eingreifen kann.

In Figur 12 ist schematisch gestrichelt ein zweites Sperrelement 170 eingezeichnet, sodass auch hier, wie bei dem in Figur 11 dargestellten Ausführungsbeispiel der Sperreinrichtung 168, wahlweise eines der beiden ersten Sperrelemente 170 mit dem Rückhaltevorsprung 204 in Eingriff gebracht werden kann, um eine Bewegung des Schiebeschafts 14 relativ zum Werkzeugelementträger 12 in proximaler Richtung zu verhindern.

Der Anschlagfläche 176 entgegen weisend ist bei dem in Figur 12 dargestellten Ausführungsbeispiels der Sperreinrichtung 168 keine korrespondierende Fläche zugeordnet, die eine Bewegung des Schiebeschafts 14 in distaler Richtung blockieren könnte, wie dies bei den Rückhaltenuten 174 der Ausführungsbeispiele der Figuren 1 bis 10 beziehungsweise 11 möglich ist.

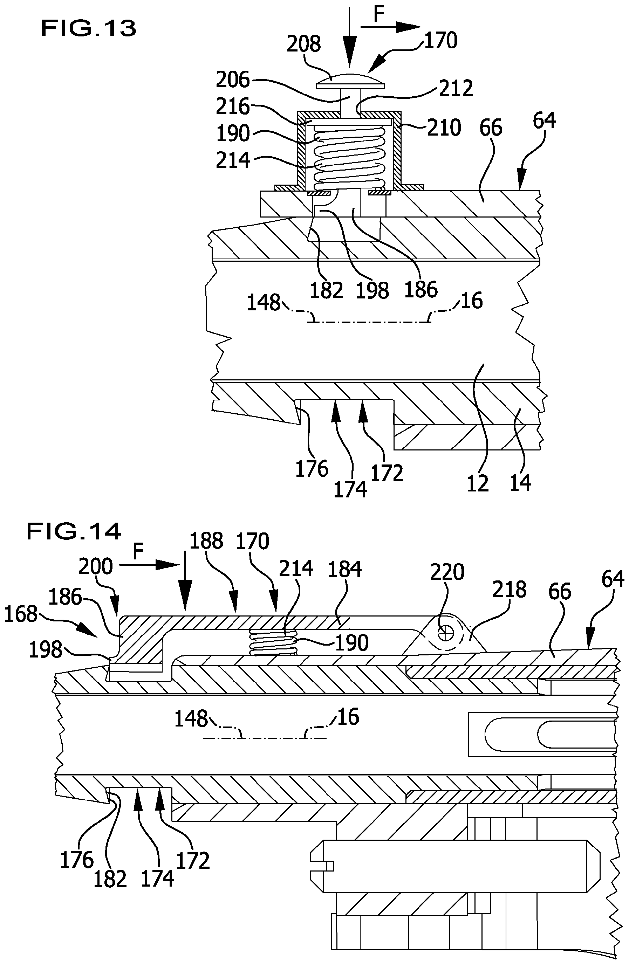

Ein weiteres Ausführungsbeispiel einer Sperreinrichtung 168 ist in Figur 13 schematisch dargestellt. Es unterscheidet sich in seinem Aufbau vom Ausführungsbeispiel der Figuren 1 bis 10 durch die Ausgestaltung des ersten Sperrelements 170. Das zweite Sperrelement 172 ist wiederum in Form einer Rückhaltenut 174 ausgebildet, welche eine Anschlagfläche 176 umfasst, die in proximaler Richtung weist und mit der Ringfläche 180 eine Hinterschneidung 182 definiert.

Das erste Sperrelement 170 umfasst einen bolzenförmigen, sich quer zur Verschieberichtung 16 erstreckenden Sperrelementabschnitt 206, an dessen vom Schiebeschaft 14 weg weisenden Ende ein Betätigungsknopf 208 angeordnet ist. Am vom Betätigungsknopf 208 weg weisenden, in Richtung auf den Schiebeschaft 14 hin weisenden Ende des Sperrelementabschnitts 206 ist ein Sperrvorsprung 186 angeordnet, mit einer in distaler Richtung weisenden Rastnase 198 zum kraft- und/oder formschlüssigen in Eingriff Bringen mit der Hinterschneidung 182.

An der Hülse 66 ist eine Haube 210 angeordnet, die eine Durchbrechung 212 für den Sperrelementabschnitt 206 aufweist.

Etwas beabstandet vom Betätigungsknopf 208 ist am Sperrelementabschnitt 206 eine Anschlagplatte 216 angeordnet, an deren in Richtung auf den Schiebeschaft 14 hin weisenden Seitenfläche einerseits und an der Hülse 66 andererseits sich ein Rückstellelement 190 in Form einer Schraubenfeder 214 abstützt und das erste Sperrelement 170 in der Freigabestellung hält.

Wird auf den Betätigungsknopf 208 eine Betätigungskraft in Richtung auf den Schiebeschaft 14 ausgeübt, wird der Sperrelementabschnitt 206, der abschnittsweise vom Rückhalteelement 190 umgeben ist, entgegen der Wirkung desselben in Richtung auf den Schiebeschaft 14 hin bewegt.

Ist der Schiebeschaft 14 so weit in distaler Richtung bewegt, dass der Sperrvorsprung 186 in die Rückhaltenut 174 eintauchen kann, kann der Schiebeschaft 14 und damit das Schiebeschaftinstrument 10 in der beschriebenen Weise in der Vorschubstellung blockiert werden.

In Figur 14 ist ein weiteres Ausführungsbeispiel einer Sperreinrichtung 168 schematisch dargestellt. Auch bei diesem Ausführungsbeispiel ist das zweite Sperrelement 172 wieder als Rückhaltenut 174 mit einer in proximaler Richtung wirkenden Hinterschneidung 182 ausgebildet.

Das erste Sperrelement 170 ähnelt in seinem Aufbau und seiner Funktionsweise dem in Zusammenhang mit den Figuren 1 bis 10 beschriebenen ersten Sperrelement 170.

An der Hülse 66 des Kopplungsabschnitts 64 ist ein Lagerbock 218 in radialer Richtung von der Längsachse 148 abstehend angeordnet, an dem um eine Schwenkachse 220 ein sich in distaler Richtung erstreckender starrer, also im Wesentlichen unverformbarer, Hebelarm 184 verschwenkbar gelagert ist. Der Hebelarm trägt an seinem in distaler Richtung weisenden Ende wiederum einen Sperrvorsprung 186, welcher vom Hebelarm 184 in Richtung auf den Schiebeschaft 14 vorstehend ausgebildet ist. Vom Sperrvorsprung 186 steht wiederum eine Rastnase 198 in distaler Richtung weisend vor, die zusammen mit der Hinterschneidung 182 eine oben eingehend beschriebene Sicherungseinrichtung 200 bildet.

Die Schwenkachse 220 verläuft quer zur Verschieberichtung 16.

Die Rückstelleinrichtung 188 umfasst ein Rückstellelement 190 in Form einer Schraubenfeder 214, die sich einerseits an der Hülse 66 und andererseits am Hebelarm 184 abstützt. Dies ermöglicht es, den Hebelarm 184 durch Beaufschlagen einer Druckkraft in Richtung auf den Schiebeschaft 14 hin zu verschwenken, und zwar entgegen der Wirkung des Rückstellelements 190.

Wie bereits oben mehrfach erläutert, kann der Sperrvorsprung 186 in die Rückhaltenut 174 eintauchen, wenn der Schiebeschaft 14 hinreichend weit in distaler Richtung aus seiner Grundstellung bewegt ist.

Wird die Sicherungseinrichtung 200 durch Bewegen des Schiebeschafts 14 in distaler Richtung entsichert, bewegt das Rückstellelement 190 den Hebelarm 184 wieder in seine Ausgangsstellung zurück, in welcher der Sperrvorsprung 186 und die Rückhaltenut 174 außer Eingriff stehen. Der Schiebeschaft 14 kann nun in distaler und proximaler Richtung innerhalb des vorgegebenen Verschiebewegs 130 bewegt werden.

Die beschriebenen Sperreinrichtungen 168 können wie erläutert im Wesentlichen beliebig mit dem beschriebenen Grundaufbau des Schiebeschaftinstruments 10 kombiniert werden. So lassen sich unterschiedliche Ausführungsbeispiele von Schiebeschaftinstrumenten 10 in Form von Clipanlegezangen 222 ausbilden.

Die beschriebenen Ausführungsbeispiele von Sperreinrichtungen 168 haben gegenüber herkömmlich bekannten Sperreinrichtungen, die aus dünnen Blechen ausgebildet sind, eine deutlich längere Lebensdauer, sind einfacher herzustellen und einfacher in der Handhabung und bieten eine erhöhte Funktionssicherheit. Diese ist insbesondere beim Einsatz des medizinischen Schiebeschaftinstruments 10 in der Neurochirurgie von großem Interesse, um insbesondere ein unbeabsichtigtes Lösen eines mit dem Schiebeschaftinstrument 10 gehaltenen Clips 30 im Kopf eines Patienten zu verhindern.

### Bezugszeichenliste

- 10: Schiebeschaftinstrument
- 12: Werkzeugelementträger
- 14: Schiebeschaft
- 16: Verschieberichtung
- 18: Betätigungseinrichtung
- 20: Werkzeugelement
- 22: Werkzeugelement
- 23: Ende
- 24: Schlitz
- 26: Haltefläche
- 28: Haltefläche
- 30: Clip
- 32: Endabschnitt
- 34: Pfeil
- 36: Außenfläche
- 38: Außenfläche
- 40: Klemmarm
- 42: Klemmarm
- 44: Federelement
- 46: Ende
- 48: Ende
- 50: Betätigungselement
- 52: Betätigungselement
- 54: Vorspannelement
- 56: Vorspannelement
- 58: Ende
- 60: Ende
- 62: Vorspanneinrichtung
- 64: Kopplungsabschnitt
- 66: Hülse
- 68: Lagerbacke
- 70: Lagerbacke
- 72: Ende
- 74: Ende
- 76: Lagerstift
- 78: Lagerstift
- 80: Schwenkachse
- 82: Schwenkachse
- 84: Mitnehmerglied
- 86: Hülsenabschnitt
- 88: Kupplungszapfen
- 90: Fenster
- 92: Schwenkachse
- 94: Lenker
- 96: Lenker
- 98: Lenkeranordnung
- 100: Lagerbacke
- 102: Lagerbacke
- 104: Lagerstift
- 106: Lagerstift
- 108: Anschlageinrichtung
- 110: Anschlag
- 112: Anschlag
- 114: Madenschraube
- 116: Madenschraube
- 118: Anschlagvorsprung
- 120: Anschlagvorsprung
- 122: Innengewinde
- 124: Innengewinde
- 126: Bohrung
- 128: Bohrung
- 130: Verschiebeweg
- 132: Abstand
- 134: Wirkfläche
- 136: Wirkfläche
- 138: Endabschnitt
- 140: Federarm
- 142: Vertiefung
- 144: Ringvorsprung
- 146: Aufgleitfläche
- 148: Längsachse
- 150: Ende
- 152: Schlitz
- 154: Federarm
- 156: Ringnut
- 158: Ringvorsprung
- 160: Verriegelungsstift
- 162: Schubglied
- 164: Element
- 166: Kappe
- 168: Sperreinrichtung
- 170: erstes Sperrelement
- 172: zweites Sperrelement
- 174: Rückhaltenut
- 176: Anschlagfläche
- 178: Neigungswinkel
- 180: Ringfläche
- 182: Hinterschneidung
- 184: Hebelarm
- 186: Sperrvorsprung
- 188: Rückstelleinrichtung
- 190: Rückstellelement
- 192: Federelement
- 194: Blattfeder
- 196: Pfeil
- 198: Rastnase
- 200: Sicherungseinrichtung
- 202: Sperrklinke
- 204: Rückhaltevorsprung
- 206: Sperrelementabschnitt
- 208: Betätigungsknopf
- 210: Haube
- 212: Durchbrechung
- 214: Schraubenfeder
- 216: Anschlagplatte
- 218: Lagerbock
- 220: Schwenkachse
- 222: Clipanlegezange

## Patentansprüche

1. Medizinisches Schiebeschaftinstrument (10) mit einem Werkzeugelementträger (12), einem rohrförmigen, den Werkzeugelementträger (12) umgebenden und eine Verschieberichtung (16) definierenden Schiebeschaft (14) und einer Betätigungseinrichtung (18) zum Bewegen des Schiebeschafts (14) relativ zum Werkzeugelementträger (12) in distaler Richtung, wobei am distalen Ende des Werkzeugelementträgers (12) mindestens ein Werkzeugelement (20, 22) angeordnet oder beweglich gelagert ist, wobei das mindestens eine Werkzeugelement (20, 22) mit einem distalen Endbereich (32) des Schiebeschafts (14) zusammenwirkend angeordnet oder ausgebildet ist derart, dass infolge einer Bewegung des Schiebeschafts (14) in distaler Richtung das mindestens eine Werkzeugelement (20, 22) bewegt wird, wobei das Schiebeschaftinstrument (10) ferner eine Sperreinrichtung (168) umfasst zum Blockieren einer Bewegung des Schiebeschafts (14) relativ zum Werkzeugelementträger (12) in mindestens einer Vorschubstellung, wobei der Schiebeschaft (14) in der mindestens einen Vorschubstellung gegenüber einer Grundstellung, in welcher der Schiebeschaft (14) relativ zum Werkzeugelementträger (12) maximal weit in proximaler Richtung verschoben ist, in distaler Richtung verschoben ist, wobei die Sperreinrichtung (168) eine Freigabestellung, in welcher der Schiebeschaft (14) relativ zum Werkzeugelementträger (12), insbesondere in proximaler Richtung, verschiebbar ist, und eine Sperrstellung definiert, in welcher eine Bewegung des Schiebeschafts (14) relativ zum Werkzeugelementträger (12) in proximaler und/oder distaler Richtung blockiert ist, wobei die Sperreinrichtung (168) mindestens ein erstes Sperrelement (170) und mindestens ein zweites Sperrelement (172) umfasst, welche in der Sperrstellung kraft- und/oder formschlüssig in Eingriff und in der Freigabestellung außer Eingriff stehen, **dadurch gekennzeichnet, dass** das mindestens eine zweite Sperrelement (172) am Schiebeschaft (14) angeordnet oder ausgebildet ist und dass das mindestens eine erste Sperrelement (170) von der Freigabestellung durch eine Bewegung auf den Schiebeschaft (14) hin in die Sperrstellung überführbar ist.

2. Medizinisches Schiebeschaftinstrument nach Anspruch 1, **dadurch gekennzeichnet, dass**
a) das mindestens eine zweite Sperrelement (172) und der Schiebeschaft (14) einstückig ausgebildet sind
und/oder
b) das mindestens eine erste Sperrelement (170)
- in einer Richtung quer, insbesondere senkrecht, zur Verschieberichtung (16) von der Freigabestellung in die Sperrstellung bewegbar ist
und/oder
- am Schiebeschaftinstrument (10) und relativ zum Werkzeugelementträger (12) in der Verschieberichtung (16) unbeweglich oder im Wesentlichen unbeweglich angeordnet oder ausgebildet ist.

3. Medizinisches Schiebeschaftinstrument nach einem der voranstehenden Ansprüche, **dadurch gekennzeichnet, dass** die Betätigungseinrichtung (18) zwei relativ zueinander bewegbare, insbesondere verschwenkbare, Betätigungselemente (50, 52) umfasst,
wobei insbesondere die zwei Betätigungselemente (50, 52)
a) bei maximal in proximaler Richtung verschobenem Schiebeschaft (14) maximal weit voneinander entfernt und zum Bewegen des Schiebeschafts (14) in distaler Richtung aufeinander zu bewegbar sind
und/oder
b) jeweils um eine Schwenkachse (80, 82) verschwenkbar sind, welche quer, insbesondere senkrecht, zur Verschieberichtung (16) verläuft.

4. Medizinisches Schiebeschaftinstrument nach einem der voranstehenden Ansprüche, **dadurch gekennzeichnet, dass** das Schiebeschaftinstrument (10) eine Vorspanneinrichtung (62) umfasst zum Ausüben einer in proximaler Richtung wirkenden vorspannenden Kraft auf den Schiebeschaft (14),
wobei insbesondere die Vorspanneinrichtung (62) mindestens ein Vorspannelement (54, 56) umfasst und dass die zwei Betätigungselemente (50, 52) entgegen der Wirkung des mindestens einen Vorspannelements (54, 56) aufeinander zu bewegbar sind.

5. Medizinisches Schiebeschaftinstrument nach einem der voranstehenden Ansprüche, **dadurch gekennzeichnet, dass** das mindestens eine zweite Sperrelement (172) eine in proximaler Richtung weisende Anschlagfläche (176) umfasst.

6. Medizinisches Schiebeschaftinstrument nach Anspruch 5, **dadurch gekennzeichnet, dass** das mindestens eine zweite Sperrelement (172) eine Hinterschneidung (182) aufweist und dass die Hinterschneidung (182) mindestens teilweise von der Anschlagfläche (176) begrenzt ist.

7. Medizinisches Schiebeschaftinstrument nach Anspruch 5 oder 6, **dadurch gekennzeichnet, dass** die Anschlagfläche (176) in Richtung auf den Schiebeschaft (14) hin weisend geneigt ist,
wobei insbesondere ein Neigungswinkel (178) zwischen der Anschlagfläche (176) und der Verschieberichtung (16) in einem Bereich von etwa 88° bis etwa 60° liegt.

8. Medizinisches Schiebeschaftinstrument nach einem der voranstehenden Ansprüche, **dadurch gekennzeichnet, dass** das mindestens eine zweite Sperrelement (172)
a) in Form eines vom Schiebeschaft (14) in radialer Richtung weisenden Rückhaltevorsprungs (204) oder in Form einer vom Schiebeschaft (14) weg weisend geöffneten Rückhaltenut (174) ausgebildet ist
und/oder
b) den Schiebeschaft (14) ringförmig umgibt, insbesondere in sich geschlossen.

9. Medizinisches Schiebeschaftinstrument nach einem der voranstehenden Ansprüche, **dadurch gekennzeichnet, dass**
a) die Sperreinrichtung (168) eine Rückstelleinrichtung (188) umfasst derart, dass das mindestens eine erste Sperrelement (170) entgegen der Wirkung der Rückstelleinrichtung (188) von der Freigabestellung in die Sperrstellung bewegbar ist,
wobei insbesondere die Rückstelleinrichtung (188) mindestens ein Rückstellelement (190) umfasst, welches mit dem mindestens einen ersten Sperrelement (170) zusammenwirkend angeordnet oder ausgebildet ist,
wobei weiter insbesondere das mindestens eine Rückstellelement (190) ein Federelement (192) umfasst, insbesondere in Form einer Schraubenfeder (214) oder einer Blattfeder (194),
und/oder
b) das mindestens eine erste Sperrelement (170) einen in Richtung auf den Schiebeschaft (14) hin weisenden Sperrvorsprung (186) umfasst, welcher in der Sperrstellung mit dem mindestens einen zweiten Sperrelement (172) in Eingriff steht.

10. Medizinisches Schiebeschaftinstrument nach einem der voranstehenden Ansprüche, **dadurch gekennzeichnet, dass** das mindestens eine erste Sperrelement (170) einen in Richtung auf den Schiebeschaft (14) hin weisenden Sperrvorsprung (186) umfasst, welcher in der Sperrstellung mit dem mindestens einen zweiten Sperrelement (172) in Eingriff steht, dass das mindestens eine zweite Sperrelement (172) eine in proximaler Richtung weisende Anschlagfläche (176) umfasst, dass das mindestens eine zweite Sperrelement (172) eine Hinterschneidung (182) aufweist, dass die Hinterschneidung (182) mindestens teilweise von der Anschlagfläche (176) begrenzt ist, dass das mindestens eine erste Sperrelement (170) eine Rastnase (198) umfasst, welche in der Sperrstellung formschlüssig oder im Wesentlichen formschlüssig in die Hinterschneidung (182) am mindestens einen zweiten Sperrelement (172) eingreift, wobei insbesondere die Rastnase (198) in distaler Richtung weisend angeordnet oder ausgebildet ist, insbesondere am Sperrvorsprung (186), wobei weiter insbesondere das mindestens eine erste Sperrelement (170) einen Hebelarm (184) umfasst und dass der Sperrvorsprung (186) an einem freien Ende des Hebelarms (184) angeordnet oder ausgebildet ist,
wobei weiter insbesondere der Hebelarm (184)
a) in distaler Richtung weisend am Schiebeschaftinstrument (10) angeordnet oder ausgebildet ist
und/oder
b) proximalseitig am Schiebeschaftinstrument (10) festgelegt ist, insbesondere um eine Hebelarmschwenkachse (220) verschwenkbar gelagert, welche Hebelarmschwenkachse (220) insbesondere quer zur Verschieberichtung (16) verläuft.

11. Medizinisches Schiebeschaftinstrument nach einem der voranstehenden Ansprüche, **dadurch gekennzeichnet, dass** das mindestens eine erste Sperrelement (170) einen quer zur Verschieberichtung (16) verlaufenden Sperrelementabschnitt (206) umfasst,
wobei insbesondere
a) das mindestens eine Rückstellelement (190) den Sperrelementabschnitt (206) umgebend angeordnet oder ausgebildet ist
und/oder
b) das mindestens eine erste Sperrelement (170) einen in Richtung auf den Schiebeschaft (14) hin weisenden Sperrvorsprung (186) umfasst, welcher in der Sperrstellung mit dem mindestens einen zweiten Sperrelement (172) in Eingriff steht, und der Sperrvorsprung (186) an einem auf den Schiebeschaft (14) hin weisenden Ende des Sperrelementabschnitts (206) angeordnet oder ausgebildet ist.

12. Medizinisches Schiebeschaftinstrument nach einem der voranstehenden Ansprüche, **dadurch gekennzeichnet, dass** das Schiebeschaftinstrument (10) eine Sicherungseinrichtung (200) umfasst zum Sichern der Sperreinrichtung (168) in der Sperrstellung,
wobei insbesondere die Sicherungseinrichtung (200)
a) das mindestens eine erste Sperrelement (170) und das mindestens eine zweite Sperrelement (172) umfasst, welche derart angeordnet oder ausgebildet sind, dass die Sperreinrichtung (168) nur durch eine Bewegung des Schiebeschafts (14) in distaler Richtung entsicherbar ist,
und/oder
b) eine Hinterschneidung (182) und die Rastnase (198) umfasst, wobei das mindestens eine zweite Sperrelement (172) eine in proximaler Richtung weisende Anschlagfläche (176) umfasst, wobei das mindestens eine zweite Sperrelement (172) die Hinterschneidung (182) aufweist und wobei die Hinterschneidung (182) mindestens teilweise von der Anschlagfläche (176) begrenzt ist.

13. Medizinisches Schiebeschaftinstrument nach einem der voranstehenden Ansprüche, **dadurch gekennzeichnet, dass**
a) das Schiebeschaftinstrument (10) eine Anschlageinrichtung (108) umfasst zum Begrenzen einer Bewegung des Schiebeschafts (14) in proximaler Richtung und in distaler Richtung,
wobei insbesondere die Anschlageinrichtung (108) einen proximalen Anschlag (110) und einen distalen Anschlag (112) umfasst und wobei ein maximaler Verschiebeweg (130) des Schiebeschafts (14) relativ zum Werkzeugelementträger (12) definiert wird durch einen Abstand von Wirkflächen (134, 136) des proximalen Anschlags (110) und des distalen Anschlags (112) voneinander,
wobei weitere insbesondere der proximale Anschlag (110) die Grundstellung und der distale Anschlag (112) eine in distaler Richtung maximal ausgelenkte Stellung des Schiebeschafts (14) definieren,
wobei weiter insbesondere die Sperreinrichtung (168) eine Bewegung des Schiebeschafts (14) relativ zum Werkzeugelementträger (12) in mindestens einer Vorschubstellung zwischen der Grundstellung und der in distaler Richtung maximal ausgelenkten Stellung blockiert,
und/oder
b) der Werkzeugelementträger (12) mit der Betätigungseinrichtung (18) in einer Arbeitsstellung gekoppelt und in einer Reinigungsstellung von der Betätigungseinrichtung (18) getrennt ist,
wobei insbesondere der Schiebeschaft (14) mit der Betätigungseinrichtung (18) in der Arbeitsstellung gekoppelt und in der Reinigungsstellung von der Betätigungseinrichtung (18) getrennt ist.

14. Medizinisches Schiebeschaftinstrument nach einem der Ansprüche 3 bis 13, **dadurch gekennzeichnet, dass** die Betätigungseinrichtung (18) einen Kopplungsteil (64) umfasst, dass die zwei Betätigungselemente (50, 52) am Kopplungsteil (64) bewegbar angeordnet sind und dass der Werkzeugelementträger (12) in der Arbeitsstellung unbeweglich am Kopplungsteil (64) gehalten ist,
wobei insbesondere
a) der Kopplungsteil (64) ein in der Verschieberichtung (16) verschiebbar gelagertes Mitnehmerglied (84) umfasst und wobei das Mitnehmerglied (84) über eine Lenkeranordnung (98) mit den zwei Betätigungselementen (50, 52) gekoppelt ist zum Bewegen des Mitnehmerglieds (84) in der Verschieberichtung (16) infolge einer Bewegung der zwei Betätigungselemente (50, 52) aufeinander zu oder voneinander weg
und/oder
b) ein proximales Ende (150) des Schiebeschafts (14) in der Arbeitsstellung mit dem Mitnehmerglied (84) gekoppelt ist
und/oder
c) das mindestens eine Rückstellelement (190) einerseits am Kopplungsteil (64) und andererseits am mindestens einen ersten Sperrelement (170), insbesondere am Hebelarm (184), angreift.

15. Medizinisches Schiebeschaftinstrument nach einem der voranstehenden Ansprüche, **dadurch gekennzeichnet, dass**
a) das mindestens eine erste Sperrelement (170) in Form einer in der Sperrstellung am Schiebeschaft (14) angreifenden Sperrklinke (202) ausgebildet ist
und/oder
b) die Verschieberichtung (16) geradlinig oder gekrümmt verläuft
und/oder
c) das mindestens eine Werkzeugelement (20, 22) und der Werkzeugelementträger (12) einstückig ausgebildet sind
und/oder
d) das Schiebeschaftinstrument (10) in Form einer Clipanlegezange (222) ausgebildet ist.

## Claims

1. Medical sliding shaft instrument (10) comprising a tool element carrier (12), a tubular sliding shaft (14) surrounding the tool element carrier (12) and defining a sliding direction (16), and an actuating device (18) for moving the sliding shaft (14) relative to the tool element carrier (12) in the distal direction, wherein at least one tool element (20, 22) is arranged or movably mounted on the distal end of the tool element carrier (12), wherein the at least one tool element (20, 22) is arranged or formed so as to cooperate with a distal end region (32) of the sliding shaft (12) in such a way that the at least one tool element (20, 22) is moved as a result of a movement of the sliding shaft (14) in the distal direction, wherein the sliding shaft instrument (10) further comprises a locking device (168) for blocking a movement of the sliding shaft (14) relative to the tool element carrier (12) in at least one advancing position, wherein the sliding shaft (14) in the at least one advancing position is displaced in the distal direction in relation to a basic position in which the sliding shaft (14) is displaced to a maximum extent in the proximal direction relative to the tool element carrier (12), wherein the locking device (168) defines a release position in which the sliding shaft (14) is displaceable, in particular in the proximal direction, relative to the tool element carrier (12), and a locking position in which a movement of the sliding shaft (14) relative to the tool element carrier (12) in the proximal and/or distal direction is blocked, wherein the locking device (168) comprises at least one first locking element (170) and at least one second locking element (172), which in the locking position are in forcelocking and/or positive-locking engagement and in the release position are out of engagement, **characterized in that** the at least one second locking element (172) is arranged or formed on the sliding shaft (14) and **in that** the at least one first locking element (170) is transferable from the release position into the locking position by a movement toward the sliding shaft (14).

2. Medical sliding shaft instrument in accordance with Claim 1, **characterized in that**
a) the at least one second locking element (172) and the sliding shaft (14) are formed in one piece
and/or
b) the at least one first locking element (170)
- is movable in a direction transverse, in particular perpendicular, to the sliding direction (16) from the release position into the locking position
and/or
- is arranged or formed on the sliding shaft instrument (10) so as to be immovable or substantially immovable relative to the tool element carrier (12) in the sliding direction (16).

3. Medical sliding shaft instrument in accordance with any one of the preceding Claims, **characterized in that** the actuating device (18) comprises two actuating elements (50, 52) that are movable, in particular pivotable, relative to one another,
wherein, in particular, the two actuating elements (50, 52),
a) when the sliding shaft (14) is displaced to a maximum extent in the proximal direction, are at a maximum distance from one another and are movable toward one another for moving the sliding shaft (14) in the distal direction
and/or
b) are each pivotable about a pivot axis (80, 82), which extends transversely, in particular perpendicularly, to the sliding direction (16).

4. Medical sliding shaft instrument in accordance with any one of the preceding Claims, **characterized in that** the sliding shaft instrument (10) comprises a biasing device (62) for exerting a biasing force acting in the proximal direction on the sliding shaft (14),
wherein, in particular, the biasing device (62) comprises at least one biasing element (54, 56) and **in that** the two actuating elements (50, 52) are movable toward one another against the action of the at least one biasing element (54, 56).

5. Medical sliding shaft instrument in accordance with any one of the preceding Claims, **characterized in that** the at least one second locking element (172) comprises a stop face (176) facing in the proximal direction.

6. Medical sliding shaft instrument in accordance with Claim 5, **characterized in that** the at least one second locking element (172) has an undercut (182) and **in that** the undercut (182) is delimited at least partially by the stop face (176).

7. Medical sliding shaft instrument in accordance with Claim 5 or 6, **characterized in that** the stop face (176) is inclined facing in the direction toward the sliding shaft (14),
wherein, in particular, an angle of inclination (178) between the stop face (176) and the sliding direction (16) is in a range of about 88° to about 60°.

8. Medical sliding shaft instrument in accordance with any one of the preceding Claims, **characterized in that** the at least one second locking element (172)
a) is configured in the form of a retaining projection (204) pointing in the radial direction from the sliding shaft (14) or in the form of a retaining groove (174) that is open facing away from the sliding shaft (14)
and/or
b) annularly surrounds the sliding shaft (14), in particular is selfenclosed.

9. Medical sliding shaft instrument in accordance with any one of the preceding Claims, **characterized in that**
a) the locking device (168) comprises a restoring device (188) in such a way that the at least one first locking element (170) is movable against the action of the restoring device (188) from the release position into the locking position,
wherein, in particular, the restoring device (188) comprises at least one restoring element (190), which is arranged or formed so as to cooperate with the at least one first locking element (170), wherein, further in particular, the at least one restoring element (190) comprises a spring element (192), in particular in the form of a coil spring (214) or a leaf spring (194),
and/or
b) the at least one first locking element (170) comprises a locking projection (186) pointing in the direction toward the sliding shaft (14), which in the locking position is in engagement with the at least one second locking element (172).

10. Medical sliding shaft instrument in accordance with any one of the preceding Claims, **characterized in that** the at least one first locking element (170) comprises a locking projection (186) pointing in the direction toward the sliding shaft (14), which in the locking position is in engagement with the at least one second locking element (172), **in that** the at least one second locking element (172) comprises a stop face (176) facing in the proximal direction, **in that** the at least one second locking element (172) has an undercut (182), **in that** the undercut (182) is delimited at least partially by the stop face (176), **in that** the at least one first locking element (170) comprises a latching nose (198), which in the locking position engages in a positive-locking or substantially positive-locking manner into the undercut (182) on the at least one second locking element (172),
wherein, in particular, the latching nose (198) is arranged or formed, in particular on the locking projection (186), pointing in the distal direction, wherein, further in particular, the at least one first locking element (170) comprises a lever arm (184) and **in that** the locking projection (186) is arranged or formed on a free end of the lever arm (184),
wherein, further in particular, the lever arm (184)
a) is arranged or formed on the sliding shaft instrument (10) pointing in the distal direction
and/or
b) is fixed to the sliding shaft instrument (10) on the proximal side, in particular mounted so as to be pivotable about a lever arm pivot axis (220), which lever arm pivot axis (220) extends, in particular, transversely to the sliding direction (16).

11. Medical sliding shaft instrument in accordance with any one of the preceding Claims, **characterized in that** the at least one first locking element (170) comprises a locking element portion (206) extending transversely to the sliding direction (16),
wherein, in particular,
a) the at least one restoring element (190) is arranged or formed surrounding the locking element portion (206)
and/or
b) the at least one first locking element (170) comprises a locking projection (186) pointing in the direction toward the sliding shaft (14), which in the locking position is in engagement with the at least one second locking element (172), and the locking projection (186) is arranged or formed on an end of the locking element portion (206) pointing toward the sliding shaft (14).

12. Medical sliding shaft instrument in accordance with any one of the preceding Claims, **characterized in that** the sliding shaft instrument (10) comprises a securing device (200) for securing the locking device (168) in the locking position,
wherein, in particular, the securing device (200)
a) comprises the at least one first locking element (170) and the at least one second locking element (172), which are arranged or formed in such a way that the locking device (168) is releasable only by a movement of the sliding shaft (14) in the distal direction,
and/or
b) comprises a undercut (182) and the latching nose (198), wherein the at least one second locking element (172) comprises a stop face (176) facing in the proximal direction, wherein the at least one second locking element (172) has the undercut (182) and wherein the undercut (182) is delimited at least partially by the stop face (176).

13. Medical sliding shaft instrument in accordance with any one of the preceding Claims, **characterized in that**
a) the sliding shaft instrument (10) comprises a stop device (108) for delimiting a movement of the sliding shaft (14) in the proximal direction and in the distal direction,
wherein, in particular, the stop device (108) comprises a proximal stop (110) and a distal stop (112) and wherein a maximum displacement path (130) of the sliding shaft (14) relative to the tool element carrier (12) is defined by a distance of active surfaces (134, 136) of the proximal stop (110) and the distal stop (112) from one another,
wherein, further in particular, the proximal stop (110) defines the basic position of the sliding shaft (14) and the distal stop (112) defines a maximally deflected position of the sliding shaft (14) in the distal direction,
wherein, further in particular, the locking device (168) blocks a movement of the sliding shaft (14) relative to the tool element carrier (12) in at least one advancing position between the basic position and the maximally deflected position in the distal direction,
and/or
b) the tool element carrier (12) is coupled to the actuating device (18) in a working position and is separated from the actuating device (18) in a cleaning position,
wherein, in particular, the sliding shaft (14) is coupled to the actuating device (18) in the working position and is separated from the actuating device (18) in the cleaning position.

14. Medical sliding shaft instrument in accordance with any one of Claims 3 to 13, **characterized in that** the actuating device (18) comprises a coupling part (64), **in that** the two actuating elements (50, 52) are movably arranged on the coupling part (64), and **in that** the tool element carrier (12) is immovably held on the coupling part (64) in the working position,
wherein, in particular,
a) the coupling part (64) comprises a driving member (84) mounted so as to be displaceable in the sliding direction (16) and wherein the driving member (84) is coupled to the two actuating elements (50, 52) by way of a connecting rod arrangement (98) for moving the driving member (84) in the sliding direction (16) as the result of a movement of the two actuating elements (50, 52) toward one another or away from one another
and/or
b) a proximal end (150) of the sliding shaft (14) is coupled to the driving member (84) in the working position
and/or
c) the at least one restoring element (190) engages on the coupling part (64) on the one hand and on the at least one first locking element (170), in particular on the lever arm (184), on the other hand.

15. Medical sliding shaft instrument in accordance with any one of the preceding Claims, **characterized in that**
a) the at least one first locking element (170) is configured in the form of a pawl (202) that engages on the sliding shaft (14) in the locking position
and/or
b) the sliding direction (16) runs rectilinearly or in a curved manner
and/or
c) the at least one tool element (20, 22) and the tool element carrier (12) are formed in one piece
and/or
d) the sliding shaft instrument (10) is configured in the form of a clip applier (222).

## Revendications

1. Instrument médical à tige coulissante (10) avec un support d'élément d'outil (12), une tige coulissante (14) tubulaire entourant le support d'élément d'outil (12) et définissant une direction de coulissement (16) et un dispositif d'actionnement (18) pour le déplacement de la tige coulissante (14) par rapport au support d'élément d'outil (12) dans la direction distale, dans lequel, à l'extrémité distale du support d'élément d'outil (12), est disposé ou logé de manière mobile au moins un élément d'outil (20, 22), dans lequel l'au moins un élément d'outil (20, 22) est disposé de façon à interagir avec une partie d'extrémité distale (32) de la tige coulissante (14) ou est conçu de sorte que, du fait d'un déplacement de la tige coulissante (14) dans la direction distale, l'au moins un élément d'outil (20, 22) est déplacé, dans lequel l'instrument médical à tige coulissante (10) comprend en outre un dispositif de blocage (168) pour le blocage d'un déplacement de la tige coulissante (14) par rapport au support d'élément d'outil (12) dans au moins une position d'avance, dans lequel la tige coulissante (14), dans l'au moins une position d'avance par rapport à une position de base, dans laquelle la tige coulissante (14) est coulissée par rapport au support d'élément d'outil (12) au maximum dans la direction proximale, est coulissé dans la direction distale, dans lequel le dispositif de blocage (168) définit une position de déblocage, dans laquelle la tige coulissante (14) peut coulisser par rapport au support d'élément d'outil (12), plus particulièrement dans la direction proximale, et une position de blocage, dans laquelle un déplacement de la tige coulissante (14) par rapport au support d'élément d'outil (12) dans la direction proximale et/ou distale est bloqué, dans lequel le dispositif de blocage (168) comprend au moins un premier élément de blocage (170) et au moins un deuxième élément de blocage (172), qui s'emboîtent par force et/ou par complémentarité de forme dans la position de blocage et qui se déboîtent dans la position de déblocage, **caractérisé en ce que** l'au moins un deuxième élément de blocage (172) est disposé ou est réalisé sur la tige coulissante (14) et **en ce que** l'au moins un premier élément de blocage (170) peut être passé de la position de déblocage à la position de blocage à l'aide d'un déplacement sur la tige coulissante (14).

2. Instrument médical à tige coulissante selon la revendication 1, **caractérisé en ce que**
a) l'au moins un deuxième élément de blocage (172) et la tige coulissante (14) sont réalisés d'une seule pièce
et/ou
b) l'au moins un premier élément de blocage (170)
- est mobile dans une direction transversale, plus particulièrement perpendiculaire, à la direction de coulissement (16) de la position de déblocage à la position de blocage
et/ou
- est disposé ou réalisé sur l'instrument médical à tige coulissante (10) et de manière fixe ou globalement fixe par rapport au support d'élément d'outil (12) dans la direction de coulissement (16).

3. Instrument médical à tige coulissante selon l'une des revendications précédentes, **caractérisé en ce que** le dispositif d'actionnement (18) comprend deux éléments d'actionnement (50, 52) mobiles, plus particulièrement pivotants, l'un par rapport à l'autre,
dans lequel, plus particulièrement, les deux éléments d'actionnement (50, 52)
a) sont éloignées au maximum l'un de l'autre lorsque la tige coulissante (14) est coulissée au maximum dans la direction proximale et peuvent être déplacés dans la direction distale l'un vers l'autre pour le déplacement de la tige coulissante (14)
et/ou
b) peuvent pivoter chacun autour d'un axe de pivotement (80, 82) qui s'étend transversalement, plus particulièrement perpendiculairement, à la direction de coulissement (16).

4. Instrument médical à tige coulissante selon l'une des revendications précédentes, **caractérisé en ce que** l'instrument médical à tige coulissante (10) comprend un dispositif de précontrainte (62) pour l'application d'une force de précontrainte agissant dans la direction proximale sur la tige coulissante (14),
dans lequel, plus particulièrement, le dispositif de précontrainte (62) comprend au moins un élément de précontrainte (54, 56) et **en ce que** les deux éléments d'actionnement (50, 52) peuvent être déplacés l'un vers l'autre contre l'action de l'au moins un élément de précontrainte (54, 56).

5. Instrument médical à tige coulissante selon l'une des revendications précédentes, **caractérisé en ce que** l'au moins un deuxième élément de blocage (172) comprend une surface de butée (176) orientée dans la direction proximale.

6. Instrument médical à tige coulissante selon la revendication 5, **caractérisé en ce que** l'au moins un deuxième élément de blocage (172) comprend une contre-dépouille (182) et **en ce que** la contre-dépouille (182) est délimitée au moins partiellement par la surface de butée (176).

7. Instrument médical à tige coulissante selon la revendication 5 ou 6, **caractérisé en ce que** la surface de butée (176) est inclinée en direction de la tige coulissante (14),
dans lequel, plus particulièrement, un angle d'inclinaison (178) entre la surface de butée (176) et la direction de coulissement (16) se trouve dans une plage entre environ 88° et environ 60°.

8. Instrument médical à tige coulissante selon l'une des revendications précédentes, **caractérisé en ce que** l'au moins un deuxième élément de blocage (172)
a) se présente sous la forme d'une saillie de retenue (204) partant de la tige coulissante (14) dans la direction radiale ou sous la forme d'une rainure de retenue (174) ouverte orientée à l'opposé de la tige coulissante (14)
et/ou
b) entoure, de manière annulaire, la tige coulissante (14), plus particulièrement est fermé sur lui-même.

9. Instrument médical à tige coulissante selon l'une des revendications précédentes, **caractérisé en ce que**
a) le dispositif de blocage (168) comprend un dispositif de réinitialisation (188), de sorte que l'au moins un premier élément de blocage (170) peut être déplacé contre l'action du dispositif de réinitialisation (188) de la position de déblocage vers la position de blocage,
dans lequel, plus particulièrement, le dispositif de réinitialisation (188) comprend au moins un élément de réinitialisation (190) qui est disposé ou réalisé de façon à interagir avec l'au moins un premier élément de blocage (170),
dans lequel, encore plus particulièrement, l'au moins un élément de réinitialisation (190) comprend un élément de ressort (192), plus particulièrement sous la forme d'un ressort hélicoïdal (214) ou d'un ressort à lames (194),
et/ou
b) l'au moins un premier élément de blocage (170) comprend une saillie de blocage (186) orientée en direction de la tige coulissante (14), qui s'emboîte, dans la position de blocage, avec l'au moins un deuxième élément de blocage (172).

10. Instrument médical à tige coulissante selon l'une des revendications précédentes, **caractérisé en ce que** l'au moins un premier élément de blocage (170) comprend une saillie de blocage (186) orientée en direction de la tige coulissante (14), qui s'emboîte, dans la direction de blocage, avec l'au moins un deuxième élément de blocage (172), **en ce que** l'au moins un deuxième élément de blocage (172) comprend une surface de butée (176) orientée dans la direction proximale, **en ce que** l'au moins un deuxième élément de blocage (172) présente une contre-dépouille (182), **en ce que** la contre-dépouille (182) est délimitée au moins partiellement par la surface de butée (176), **en ce que** l'au moins un premier élément de blocage (170) comprend un embout d'encliquetage (198) qui, dans la position de blocage, s'emboîte, par complémentarité de forme ou globalement par complémentarité de forme, dans la contre-dépouille (182) sur l'au moins un deuxième élément de blocage (172),
dans lequel, plus particulièrement, l'embout d'encliquetage (198) est disposé ou réalisé de manière orientée dans la direction distale, plus particulièrement sur la saillie de blocage (186),
dans lequel, encore plus particulièrement, l'au moins un premier élément de blocage (170) comprend un bras de levier (184) et **en ce que** la saillie de blocage (186) est disposée ou réalisée sur une extrémité libre du bras de levier (184),
dans lequel, encore plus particulièrement, le bras de levier (184)
a) est disposé ou réalisé sur l'instrument médical à tige coulissante (10), de façon à être orienté dans la direction distale
et/ou
b) est fixé côté proximal à l'instrument médical à tige coulissante (10), plus particulièrement logé de manière pivotante autour d'un axe de pivotement de bras de levier (220), cet axe de pivotement de bras de levier (220) s'étendant plus particulièrement transversalement par rapport à la direction de coulissement (16).

11. Instrument médical à tige coulissante selon l'une des revendications précédentes, **caractérisé en ce que** l'au moins un premier élément de blocage (170) comprend une section d'élément de blocage (206) s'étendant transversalement par rapport à la direction de coulissement (16),
dans lequel plus particulièrement
a) l'au moins un élément de réinitialisation (190) est disposé ou réalisé de façon à entourer la section d'élément de blocage (206)
et/ou
b) l'au moins un premier élément de blocage (170) comprend une saillie de blocage (186) orientée en direction de la tige coulissante (14), qui, dans la position de blocage, s'emboîte avec l'au moins un deuxième élément de blocage (172) et la saillie de blocage (186) est disposée ou réalisée sur une extrémité de la section d'élément de blocage (206), orientée vers la tige coulissante (14).

12. Instrument médical à tige coulissante selon l'une des revendications précédentes, **caractérisé en ce que** l'instrument médical à tige coulissante (10) comprend un dispositif de sécurité (200) pour la sécurisation du dispositif de blocage (168) dans la position de blocage, dans lequel, plus particulièrement, le dispositif de sécurisation (200)
a) comprend l'au moins un premier élément de blocage (170) et l'au moins un deuxième élément de blocage (172), qui sont disposés ou réalisés de sorte que le dispositif de blocage (168) ne peut être débloqué que par un mouvement de la tige coulissante (14) dans la direction distale,
et/ou
b) comprend une contre-dépouille (182) et l'embout d'encliquetage (198), dans lequel l'au moins un deuxième élément de blocage (172) comprend une surface de butée (176) orientée dans la direction proximale, dans lequel l'au moins un deuxième élément de blocage (172) présente la contre-dépouille (182) et dans lequel la contre-dépouille (182) est délimitée au moins partiellement par la surface de butée (176).

13. Instrument médical à tige coulissante selon l'une des revendications précédentes, **caractérisé en ce que**
a) l'instrument médical à tige coulissante (10) comprend un dispositif de butée (108) pour la limitation d'un déplacement de la tige coulissante (14) dans la direction proximale et dans la direction distale,
dans lequel, plus particulièrement, le dispositif de butée (108) comprend une butée proximale (110) et une butée distale (112) et dans lequel une course de coulissement maximale (130) de la tige coulissante (14) par rapport au support d'élément d'outil (12) est définie par une distance des surfaces d'action (134, 136) de la butée proximale (110) et de la butée distale (112) entre elles, dans lequel, encore plus particulièrement, la butée proximale (110) définit la position de base et la butée distale (112) définit une position, déviée au maximum dans la direction distale, de la tige coulissante (14),
dans lequel, encore plus particulièrement, le dispositif de blocage (168) bloque un déplacement de la tige coulissante (14) par rapport au support d'élément d'outil (12) dans au moins une position d'avance entre la position de base et la position déviée au maximum dans la direction distale,
et/ou
b) le support d'élément d'outil (12) est couplé avec le dispositif d'actionnement (18) dans une position de travail et est séparé du dispositif d'actionnement (18) dans une position de nettoyage
dans lequel, plus particulièrement, la tige coulissante (14) est couplée avec le dispositif d'actionnement (18) dans la position de travail et est séparée du dispositif d'actionnement (18) dans la position de nettoyage.

14. Instrument médical à tige coulissante selon l'une des revendications 3 à 13, **caractérisé en ce que** le dispositif d'actionnement (18) comprend une partie de couplage (64), **en ce que** les deux éléments d'actionnement (50, 52) sont disposés de manière mobile sur la partie de couplage (64) et **en ce que** le support d'élément d'outil (12) est maintenu de manière fixe sur la partie de couplage (64) dans la position de travail,
dans lequel, plus particulièrement,
a) la partie de couplage (64) comprend un organe d'entraînement (84) logé de manière coulissante dans la direction de coulissement (16) et dans lequel l'organe d'entraînement (84) est couplé avec les deux éléments d'actionnement (50, 52) au moyen d'une disposition de guidage (98), pour le déplacement de l'organe d'entraînement (84) dans la direction de coulissement (16) à la suite d'un déplacement des deux éléments d'actionnement (50, 52) l'un vers l'autre ou l'un loin de l'autre,
et/ou
b) une extrémité proximale (150) de la tige coulissante (14) est couplée avec l'organe d'entraînement (84) dans la position de travail
et/ou
c) l'au moins un élément de réinitialisation (190) entre en contact d'un côté avec la partie de couplage (64) et de l'autre côté avec l'au moins un premier élément de blocage (170), plus particulièrement le bras de levier (184).

15. Instrument médical à tige coulissante selon l'une des revendications précédentes, **caractérisé en ce que**
a) l'au moins un premier élément de blocage (170) se présente sous la forme d'un cliquet de blocage (202), qui entre en contact avec la tige coulissante (14) dans la position de blocage,
et/ou
b) la direction de coulissement (16) est en ligne droite ou est incurvée et/ou
c) l'au moins un élément d'outil (20, 22) et le support d'élément d'outil (12) sont réalisés d'une seule pièce
et/ou
d) l'instrument médical à tige coulissante (10) se présente sous la forme d'une pince d'appui à clip (222).
